# EUROPÄISCHE PATENTSCHRIFT

(11) **EP 3 177 267 B1**
(45) Veröffentlichungstag und Bekanntmachung des Hinweises auf die Patenterteilung: **05.01.2022**
(21) Anmeldenummer: 15712893.5
(22) Anmeldetag: 26.03.2015
(51) Int. Cl.: A61Q 17/04, A61Q 3/02, A61K 8/87, A61K 8/34

(54) **POLYURETHANHARNSTOFFLÖSUNGEN FÜR KOSMETISCHE ZUSAMMENSETZUNGEN**
POLYURETHANEUREA SOLUTIONS FOR COSMETIC COMPOSITIONS
SOLUTIONS DE POLYURÉTHANE-URÉE POUR COMPOSITIONS COSMÉTIQUES

(30) Priorität: 05.08.2014 EP 14179782
(43) Veröffentlichungstag der Anmeldung: 14.06.2017
(73) Patentinhaber: Covestro Deutschland AG, 51373 Leverkusen (DE)
(72) Erfinder: DÖRR, Sebastian, 40593 Düsseldorf (DE); VIALA, Sophie, 50935 Köln (DE); RODRIGUES, Paula Cristina Alves, 40627 Düsseldorf (DE)
(74) Vertreter: Levpat
(86) Internationale Anmeldenummer: PCT/EP2015/056579
(87) Internationale Veröffentlichungsnummer: WO 2015/075281

(56) Entgegenhaltungen:
- EP-A1- 2 105 124
- WO-A1-2007/068699
- WO-A1-2009/118103
- WO-A1-2009/118105
- WO-A1-2014/095164
- WO-A2-2011/107462
- DE-A1- 4 241 118

## Beschreibung

Die vorliegende Erfindung betrifft ein Verfahren zur Herstellung einer kosmetischen Zusammensetzung unter Verwendung von Polyurethanharnstoffen, sowie eine kosmetische Zusammensetzung erhältlich nach dem erfindungsgemäßen Verfahren und ein Verfahren zur Herstellung einer kosmetischen Beschichtung auf Haut, Nägeln und/oder keratinen Fasern unter Verwendung der erfindungsgemäßen kosmetischen Zusammensetzungen. Des Weiteren betrifft die Erfindung eine Sonnenschutz-Zusammensetzung enthaltend einen Polyurethanharnstoff und diese Sonnenschutzzusammensetzung zum Schutz von Haut und/oder Haaren vor negativen Einflüssen von Sonnenstrahlung.

Kosmetische Produkte, die hauptsächlich auf leicht verdunstenden Alkoholen, wie beispielsweise Ethanol, basieren erfreuen sich im Markt zunehmender Beliebtheit. Durch das schnelle Verdunsten des Alkohols sind die Zusammensetzungen nach dem Aufbringen auf den Körper schnell trocken und damit rasch einsatzbereit. Zudem hinterlassen insbesondere Produkte zum Auftragen auf die Haut einen erfrischend-kühlenden sensorischen Eindruck und zeigen auch nur eine sehr geringe Klebrigkeit des gebildeten Films auf der Haut. Alkohol basierte Zusammensetzungen werden bevorzugt auch zur Herstellung von transparenten Sonnensprays oder -gelen eingesetzt. Da transparente Sprays und Gele keine sichtbaren, UV-absorbierenden Partikel enthalten sollten, ist man jedoch in solchen Sonnenschutzmitteln bei der Auswahl der UV-Filter eingeschränkt und es werden eine Vielzahl öllöslicher UV-Filter verwendet. Alkohol basierte, transparente Sonnenspray werden beispielsweise in der WO 2007/068699 A1 und der DE 20 2010 006 005 U1 beschrieben.

Kosmetische Produkte zum Auftragen auf den Körper enthalten meist einen Filmbildner auf Polymerbasis. Als gute filmbildende Polymere für kosmetische Zusammensetzungen sind unter anderem wässriger Dispersionen von Polyurethanharnstoffen bekannt, wie sie beispielsweise in der WO 2009/118105 A1, WO 2009/118103 A1, WO 2011/107462 A1 WO 2012/130683 A1 und WO 2014/095164 A1 beschrieben werden. Kosmetische Zusammensetzungen, enthaltend die beschriebenen wässrigen Polyurethanharnstoffdispersionen weisen einige Vorteile auf, wie beispielsweise bei Sonnenschutz-Zusammensetzungen einen hohen SPF-Boosting Effekt (Verstärkungseffekt des Lichtschutzfaktors) und damit eine reduzierte Menge an Sonnenschutzfilter-Substanzen, die eingesetzt werden müssen um einen bestimmten hohen SPF (Lichtschutzfaktor) zu erhalten. auf. Allerdings weisen die bekannten wässrigen Dispersionen der Polyurethanharnstoffe einige Nachteile in kosmetischen Zusammensetzungen auf, die überwiegend auf alkoholischen Lösemitteln basieren. Sie führen beispielsweise zu einer Trübung der kosmetischen Produkte, insbesondere in solchen Produkten, die UV-Filter enthalten, die ausschließlich öllöslich sind. Dies wird für viele Anwendungen als nachteilig empfunden. Der zusätzliche Anteil an Wasser, der durch die wässrigen Dispersionen zusätzlich in das Produkt eingebracht wird kann zudem in einer erhöhten Klebrigkeit und verlängerten Trocknungszeit der kosmetischen Produkte resultieren. Nachteilig ist auch, dass die Polyurethanharnstoffe nach dem Stand der Technik hydrophilierende, insbesondere ionisch hydrophilierende Gruppen aufweisen, welche durch teure Verbindungen, die diese Gruppen tragen in die Polymere eingebracht werden.

DE 4242118 A1 offenbart die Verwendung von kationischen Polyurethanen und Polyharnstoffen aus a) mindestens einem Diisocyanat, welches bereits vorher mit einer oder mehreren Verbindungen, die zwei oder mehrere aktive Wasserstoffatome pro Molekül enthalten, umgesetzt sein kann, und b) mindestens einem ein oder mehrere tertiäre, quartäre oder protonierte tertiäre Aminstickstoffatome enthaltenden Diol, primären oder sekundären Aminoalkohol, primären oder sekundären Diamin oder primären oder sekundären Triamin mit einer Glasübergangstemperatur von mindestens 25°C und einer Aminzahl von 50 bis 200.

WO 2009/11805 A1 offenbart ein Verfahren zur Herstellung von VOC-armen, insbesondere wässrigen, Polyurethan-Dispersionen. Es wird ein auf einem Polyetherpolyol basierendes Polyurethan in Aceton hergestellt. Anschließend wird Wasser zugegeben und das organische Lösemittel im Vakuum entfernt, so dass eine wässrige Dispersion erhalten wird. Rein alkoholische Lösungen gemäß der vorliegenden Erfindung werden in dieser Schrift nicht offenbart.

WO 2009/118 103 A1 offenbart eine Sonnenschutz-Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B).

Ionisch hydrophilierende Gruppen tragende Polyurethanharnstoffe bilden zudem im allgemeinen keine klaren Lösungen in Alkoholen, wodurch sie für den Einsatz in transparenten, kosmetischen Zusammensetzungen weniger geeignet sind.

Solche hydrophilierenden Gruppen, insbesondere ionisch hydrophilierende Gruppen tragende Polyurethanfilmbildner werden beispielsweise auch in der DE 4241118 A1 beschrieben.

Allgemein ist bekannt, dass Polyurethanharnstoffe auf Grund ihrer Struktur zu Ausfällung bzw. Kristallisation aus organischer Lösungen neigen. Daher ist es problematisch organische Lösungen von Polyurethanharnstoffen herzustellen, die eine ausreichend hohe Molekulargewicht aufweisen, ohne dass die Polyurethanharnstoffe aus den Lösemitteln ausfallen und somit keine klaren, lagerstabilen Lösungen erhalten werden.

Zur Verhinderung dieser Kristallisation werden Lösemittelgemische empfohlen; die Lösemittel, welche in der Zwischenzeit aufgrund der gewachsenen toxikologischen Erkenntnisse zu den bedenklichen Lösemitteln gezählt werden, enthalten, wie beispielsweise Toluol, oder Xylol.

Der Einsatz derartiger Colöser ist jedoch für Polyurethanharnstoffe zur Verwendung in kosmetischen Produkten bereits aus Zulassungsrechtlichen Gesichtspunkten nicht möglich. Weiterhin weisen die in der WO 2007/068699 A1 und der DE 20 2010 006 005 U1 eingesetzten Filmbildner weisen im Gegensatz zu Polyurethanharnstoffdispersionen nur einen geringen SPF Boosting Effekt auf, so dass der Einsatz von hohen Mengen an Sonnenschutzfilter-Substanzen erforderlich ist um einen hohen SPF zu erzielen.

Aufgabe der vorliegenden Erfindung war es daher ein Verfahren zur Herstellung von kosmetischen Zusammensetzungen bereitzustellen, das es ermöglicht kosteneffiziente Polyurethanharnstoffe als Filmbildner auch in alkoholischen, kosmetischen Formulierungen, insbesondere solchen, die öllösliche UV-Filter enthalten einzusetzen, ohne dass diese eine störende Trübung aufweisen oder zwangsweise einen erhöhten Wasseranteil enthalten. Die erhaltenen kosmetischen Formulierungen sollen dabei dennoch die aus dem Stand der Technik bekannten Vorteile von wässrige Polyurethanharnstoffdispersionen in kosmetischen Formulierungen, wie beispielsweise einen SPF-Boosting Effekt bei Sonnenschutz-Zusammensetzungen, aufweisen. Bevorzugt sollte die hergestellte kosmetische Zusammensetzung geeignet sein zur Behandlung von Nägeln, Haut und/oder keratinen Fasern, besonders bevorzugt zur Behandlung von Haut und/oder Haaren.

Diese Aufgabe wurde erfindungsgemäß gelöst durch ein Verfahren zur Herstellung einer kosmetischen Zusammensetzung, wobei wenigstens ein Polyurethanharnstoff, welcher keine ionisch hydrophilierenden Gruppen aufweist und welcher gelöst in einem Lösemittel oder Lösemittelgemisch ist, eingesetzt wird, wobei das Lösemittel ausschließlich aus einem oder mehreren monohydroxyfunktionellen Alkoholen besteht oder ein Lösemittelgemisch ist, bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, die kosmetische Zusammensetzung einen Wasseranteil von ≥ 0 und ≤ 5 Gew.-% aufweist und wenigstens eine öllösliche organische Sonnenschutzfilter-Substanz enthält,
wobei der Polyurethanharnstoff aufgebaut ist aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Überraschenderweise wurde gefunden, dass durch das erfindungsgemäße Verfahren alkoholische, kosmetische Zusammensetzungen bereitgestellt werden können, die Polyurethanharnstoffe als Filmbildner enthalten, ohne dass die erhaltenen kosmetischen Zusammensetzungen eine störende Trübung aufweisen oder einen erhöhten Wasseranteil enthalten. Auch kosmetische Formulierungen enthalten UV-Filter, die gelöst in Öl vorliegen zeigen keine störende Trübung. Die kosmetischen Formulierungen weisen dennoch die aus dem Stand der Technik bekannten Vorteile von wässrigen Polyurethanharnstoffdispersionen in kosmetischen Formulierungen, wie beispielsweise einen SPF-Boosting Effekt bei Sonnenschutz-Zusammensetzungen, auf.

Ein weiterer Gegenstand der vorliegenden Erfindung ist eine kosmetische Zusammensetzung erhältlich nach dem erfindungsgemäßen Verfahren, wobei die kosmetische Zusammensetzung eine alkoholische Lösung ist, einen Wasseranteil von ≥ 0 und ≤ 5 Gew.-% aufweist und wenigstens eine öllösliche organische_Sonnenschutzfilter-Substanz enthält.

Ein weiterer Gegenstand der vorliegenden Erfindung ist ein Verfahren zur Herstellung einer kosmetischen Beschichtung auf Nägeln und/oder keratinen Fasern unter Verwendung von erfindungsgemäßen kosmetischen Zusammensetzungen, wobei die kosmetische Zusammensetzung auf Nägel und/oder keratinen Fasern aufgetragen wird.

Ein weiterer Gegenstand der vorliegenden Erfindung ist eine Sonnenschutz-Zusammensetzung enthaltend wenigstens eine öllösliche organische Sonnenschutzfiltersubstanz und wenigstens einen Polyurethanharnstoff, welcher keine ionisch hydrophilierenden Gruppen aufweist und welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch ist, bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, wobei die Zusammensetzung einen Wasseranteil von ≥ 0 und ≤ 5 Gew.-% aufweist und der Polyurethanharnstoff aufgebaut ist aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Der erfindungsgemäß verwendete, gelöste Polyurethanharnstoff, inklusive das Lösemittel oder Lösemittelgemisch, wird im Folgenden auch als Polyurethanharnstofflösung bezeichnet.

Gelöst bedeutet im Sinne der Erfindung, dass es sich bei 23°C um homogene, einphasige flüssige Gemische aus wenigstens zwei Stoffen handelt, die klar sind. Klar bedeutet im Sinne der vorliegenden Erfindung, dass die Trübungswerte der Lösung ≤ 200 NTU (Nephelometric Turbidity Unit), bevorzugt ≤ 50 NTU, besonders bevorzugt ≤ 10 NTU und ganz besonders bevorzugt ≤ 3 NTU sind. Die Trübungswerte werden dabei durch eine Streulichtmessung im 90°-Winkel (Nephelometrie) bei einer Wellenlänge der Mess-Strahlung 860 nm entsprechend DIN EN ISO 7027 bestimmt, durchgeführt bei 23°C mit einem Labortrübungsmessgerät Modell 2100AN der Firma HACH LANGE GmbH, Berlin, Deutschland.

Polyurethanharnstoffe im Sinne der Erfindung sind polymere Verbindungen, die mindestens zwei bevorzugt mindestens drei Urethan-Gruppen-haltige Wiederholungseinheiten 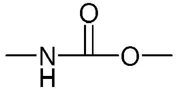 und zudem auch Harnstoff-Gruppen-haltige Wiederholungseinheiten aufweisen:

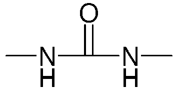

Ionisch hydrophilierende Gruppen im Sinne der Erfindung sind solche die in den Polyurethanharnstoff beispielsweise durch geeignete anionisch bzw. potentiell anionisch hydrophilierenden Verbindungen eingebracht werden könnten, die mindestens eine isocyanatreaktive Gruppe, wie eine Hydroxylgruppe oder Aminogruppe aufweisen sowie mindestens eine Funktionalität wie z.B. -COO-M⁺, -SO₃-M⁺, -PO(O-M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist.

Unter potentiell anionischen (und auch allgemein potentiell ionischen) Gruppen sind im Rahmen dieser Erfindung solche Gruppen zu verstehen, die durch Neutralisation in eine anionische (ionische) Gruppe überführt werden können.

Gemäß dem erfindungsgemäßen Verfahren weist der eingesetzte Polyurethanharnstoff keine hydrophilierenden Gruppen, also weder ionische, noch nicht-ionische hydrophilierende Gruppen auf.

Nicht-ionisch hydrophilierende Gruppen im Sinne der Erfindung sind solche, die in den Polyurethanharnstoff beispielsweise durch geeignete nicht-ionisch hydrophilierende Verbindungen eingeführt werden könnten, wie beispielsweise Polyoxyalkylenether, die mindestens eine Hydroxy- oder Aminogruppe enthalten. Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38 beschrieben). Diese Verbindungen sind entweder reine Polyethylenoxidether oder gemischte Polyalkylenoxidether, wobei sie dann aber mindestens 30 mol-%, bevorzugt mindestens 40 mol-%, bezogen auf alle enthaltenen Alkylenoxideinheiten an Ethylenoxideinheiten, enthalten.

Die Polyurethanharnstoffe der vorliegenden Erfindung werden im erfindungsgemäßen Verfahren zur Herstellung der kosmetischen Zusammensetzungen gelöst in einem Lösemittel oder Lösemittelgemisch, und somit als Polyurethanharnstofflösungen und nicht als wässrige Dispersion, eingesetzt.

Der erfindungsgemäß verwendete Polyurethanharnstoff aufgebaut aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Das zahlenmittlere Molekulargewicht wird im Rahmen dieser Anmeldung stets bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2xPSS SDV linear M, 8x300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Weiterhin bevorzugt ist der Polyurethanharnstoff aufgebaut aus ≥ 5 und ≤ 60 Gew.-% der Komponente a), ≥ 30 und ≤ 90 Gew.-% der Komponente b), ≥ 2 und ≤ 25 Gew.-% der Komponente c), ≥ 0 und ≤ 10 Gew.-% der Komponente d), ≥ 0 und ≤ 10 Gew.-% der Komponente e) und ≥ 0 und ≤ 20 Gew.-% der Komponente f), jeweils bezogen auf die Gesamtmasse des Polyurethanharnstoffs, wobei sich die Komponenten a) bis f) zu 100 Gew.-% ergänzen.

Besonders bevorzugt ist der Polyurethanharnstoff aufgebaut aus ≥ 10 und ≤ 40 Gew.-% der Komponente a), ≥ 55 und ≤ 85 Gew.-% der Komponente b), ≥ 5 und ≤ 20 Gew.-% der Komponente c), ≥ 0 und ≤ 3 Gew.-% der Komponente d), ≥ 0 und ≤ 3 Gew.-% der Komponente e) und ≥ 0 und ≤ 1 Gew.-% der Komponente f), jeweils bezogen auf die Gesamtmasse des Polyurethanharnstoffs, wobei sich die Komponenten a) bis f) zu 100 Gew.-% ergänzen.

Als Komponente a) geeignete Verbindungen sind beispielsweise 1,4-Butylendiisocyanat, 1,5-Pentamethy¬lendiisocyanat (PDI), 1,6-Hexamethy-lendiisocyanat (HDI), Isophorondiisocyanat (IPDI), 2,2,4- und/oder 2,4,4-Trimethyl-hexa¬methy¬len¬¬diiso¬cyanat, die isomeren Bis-(4,4'-isocyanatocyclo¬hexyl)¬methane oder deren Mischungen beliebigen Isomeren¬gehalts (H12-MDI), 1,4-Cyclohexylendi-isocyanat, 4-Isocyanatomethyl-1,8-octandiisocyanat (Nonantriisocyanat), 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin¬diisocyanate) mit C1-C8-Alkylgruppen.

Neben den vorstehend genannten Polyisocyanaten können anteilig auch modifizierte Diisocyanate oder Triisocyanate mit Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit einer mittleren NCO-Funktionalität von ≥ 2 und ≤ 4, bevorzugt ≥ 2 und ≤ 2,6 und besonders bevorzugt ≥ 2 und ≤ 2,4.

Bevorzugt ist die Komponente a) ausgewählt aus aliphatischen, araliphatischen und/oder cycloaliphatischen Diisocyanaten, welche mindestens eine Isocyanatgruppe aufweisen, die an ein sekundäres und/oder tertiäres C-Atom gebunden ist.

Besonders bevorzugt ist die Komponente a) ausgewählt aus IPDI und/oder H12-MDI.

Weiterhin bevorzugt werden zur Herstellung des Polyurethanharnstoffs keine aromatischen Polyisocyanate eingesetzt.

Die Komponente a) wird bevorzugt in Mengen von ≥ 5 und ≤ 60 Gew.-%, besonders bevorzugt ≥ 10 und ≤ 40 Gew.-% und ganz besonders bevorzugt von ≥ 15 und ≤ 35 Gew.-%, bezogen auf das Gesamtgewicht des Polyurethanharnstoffs, eingesetzt.

Die Komponente b) besteht aus einem oder mehreren Polyetherpolyolen mit einem zahlenmittleren Molekulargewicht Mn ≥ 400 und ≤ 6000 g/mol und einer Hydroxylfunktionalität von ≥ 1,5 und ≤ 4, bevorzugt mit einem zahlenmittleren Molekulargewicht Mn ≥ 500 und ≤ 2500 g/mol und einer Hydroxylfunktionalität von ≥ 1,9 und ≤ 3 und besonders bevorzugt mit einem zahlenmittleren Molekulargewicht Mn ≥ 1000 und ≤ 2000 g/mol und einer Hydroxylfunktionalität von ≥ 1,9 und ≤ 2,1.

Geeignete Polyetherpolyole der Komponente b) sind beispielsweise die in der Polyurethanchemie an sich bekannten Poly(tetramethylenglykol)polyetherpolyole, wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Die Polyetherpolyole weisen dabei bevorzugt einen Anteil an Gruppen erhalten aus Ethylenoxid von < 50 Gew.-%, bevorzugt < 30 Gew.-% auf.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Bevorzugt ist die Komponente b) ausgewählt aus Polypropylenglykolen und/oder Poly(tetramethylenglykol)polyetherpolyolen, besonders bevorzugt ausgewählt aus Poly(tetramethylenglykol)polyetherpolyolen.

In einer bevorzugten Ausführungsform der Erfindung handelt es sich bei der Komponente b) um ein oder mehrere Poly(tetramethylenglykol)polyetherpolyole mit einem mit einem zahlenmittleren Molekulargewicht Mn ≥ 500 und ≤ 2500 g/mol und einer Hydroxylfunktionalität von ≥ 1,9 und ≤ 2,1.

In einer besonders bevorzugten Ausführungsform ist die Komponente b) ein Gemisch aus Poly(tetramethylenglykol)polyetherpolyolen I mit einem zahlenmittleren Molekulargewichte Mₙ von ≥ 400 und ≤ 1500 g/mol, besonders bevorzugt von ≥ 600 und ≤ 1200 g/mol, ganz besonders bevorzugt von 1000 g/mol und Poly(tetramethylenglykol)polyetherpolyolen II mit einem zahlenmittleren Molekulargewichte Mₙ von ≥ 1500 und ≤ 8000 g/mol, besonders bevorzugt von ≥ 1800 und ≤ 3000 g/mol, ganz besonders bevorzugt von 2000 g/mol.

Das Gewichtsverhältnis der Poly(tetramethylenglykol)polyetherpolyole I zu den Poly(tetramethylenglykol)polyetherpolyolen II liegt bevorzugt im Bereich von ≥ 0,1 und ≤ 10, besonders bevorzugt im Bereich von ≥ 0,2 und ≤ 8, ganz besonders bevorzugt im Bereich von ≥ 1 und ≤ 6.

Die Komponente b) wird bevorzugt in Mengen von ≥ 30 und ≤ 90 Gew.-%, besonders bevorzugt ≥ 50 und ≤ 85 Gew.-%, ganz besonders bevorzugt ≥ 55 und ≤ 75 Gew.-% bezogen auf das Gesamtgewicht des Polyurethanharnstoffs, eingesetzt.

Bei der Komponente c) handelt es sich um eine oder mehrere aminofunktionelle Verbindungen, die mindestens zwei isocyanatreaktive Aminogruppen aufweisen.

Als Komponente c) sind beispielsweise geeignet Di- oder Polyamine wie 1,2-Ethylendiamin, 1,2- und 1,3-Diaminopropan, 1,4-Diaminobutan, 1,6-Diaminohexan, 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4'-Diaminodicyclohexylmethan (H12-MDA), Isophorondiamin (IPDA) und/oder 1,2-Dimethylethylendiamin.

Bevorzugt ist die Komponente c) ausgewählt aus Ethylendiamin, IPDA und/oder H12-MDA, besonders bevorzugt aus Isophorondiamin und/oder H12-MDA und ganz besonders bevorzugt ist die Komponente c) H12-MDA.

Die Verbindungen der Komponente c) enthalten bevorzugt keine hydrophilierenden Gruppen, insbesondere keine ionisch oder potentiell ionisch hydrophilierenden Gruppen.

In einer besonders bevorzugten Ausführungsform der Erfindung ist die Komponente c) ausgewählt aus Aminen, die mindestens zwei isocyanatreaktive Aminogruppen aufweisen, die an primäre und/oder sekundäre C-Atome gebunden sind.

Weiterhin bevorzugt ist die Komponente c) ausgewählt aus symmetrisch aufgebauten Diaminen. Ganz besonders bevorzugt ist die Komponente c) ausgewählt aus symmetrischen Diaminen, die mindestens zwei Aminogruppen aufweisen, die an primäre und/oder sekundäre C-Atome gebunden sind, insbesondere bevorzugt ist die Komponente c) H12-MDA.

Die Komponente c) wird bevorzugt in Mengen von ≥ 2 und ≤ 25 Gew.-%, besonders bevorzugt ≥ 5 und ≤ 20 Gew.-% und ganz besonders bevorzugt ≥ 9 und ≤ 16 Gew.-%, bezogen auf das Gesamtgewicht des Polyurethanharnstoffs, eingesetzt.

In einer bevorzugten Ausführungsform der Erfindung ist entweder die Komponente a) H12-MDI oder die Komponente c) H12-MDA oder die Komponente a) H12-MDI und die Komponente c) H12-MDA.

Gegebenenfalls wird der Polyurethanharnstoff zudem aufgebaut aus Komponente d), ein oder mehrere Alkohole, die mindestens zwei Hydroxylgruppen und eine Molmasse von ≥ 60 und ≤ 399 g/mol aufweisen, wie beispielsweise Polyole des genannten Molmassenbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit.

Die Komponente d) wird bevorzugt in Mengen von ≥ 0 und ≤ 10 Gew.-%, besonders bevorzugt ≥ 0 und ≤ 3 Gew.-%, bezogen auf das Gesamtgewicht des Polyurethanharnstoffs, und ganz besonders bevorzugt gar nicht eingesetzt.

Weiterhin kann der Polyurethanharnstoff aufgebaut sein aus Komponente e), einer oder mehreren Verbindungen, die eine gegenüber Isocyanatgruppen reaktive Gruppe aufweisen, insbesondere Verbindungen die eine Amino- oder Hydroxygruppe aufweisen. Geeignete Verbindungen der Komponente e) sind beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Methanol, Ethanol, iso-Propanol, n-Propanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Komponente e) umfasst bevorzugt keine monofunktionellen Polyetherpolyole, die einen Anteil an Gruppen erhalten aus Ethylenoxid von > 30 Gew.-%, bevorzugt > 50 Gew.-% aufweisen.

Der als Lösemittel für den Polyurethanharnstoff eingesetzte monohydroxyfunktionelle Alkohol kann ebenfalls als Aufbaukomponente e) für den Polyurethanharnstoff dienen.

Die Komponente e) wird, bevorzugt in Mengen, von ≥ 0 und ≤ 10 Gew.-%, besonders bevorzugt ≥ 0 und ≤ 3 Gew.-%, bezogen auf das Gesamtgewicht des Polyurethanharnstoffs, und ganz besonders bevorzugt gar nicht eingesetzt, wobei hier der als Lösemittel für den Polyurethanharnstoff eingesetzte monohydroxyfunktionelle Alkohol als Komponente e) unberücksichtigt bleibt.

Der monohydroxyfunktionelle Alkohol, welcher als Lösemittel für den Polyurethanharnstoff dient, macht bevorzugt ≥ 0 und ≤ 5 Gew.-%, besonders bevorzugt ≥ 0,01 und ≤ 3 Gew.-% und ganz besonders bevorzugt ≥ 0,01 und ≤ 2 Gew.-% der Gesamtmasse des Polyurethanharnstoffs aus.

Gegebenenfalls kann der Polyurethanharnstoff auch aufgebaut sein aus der Komponente f), einem Polyol oder mehreren Polyolen mit einem zahlenmittleren Molekulargewicht Mn ≥ 500 und ≤ 6000 g/mol und einer Hydroxylfunktionalität von ≥ 1,5 und ≤ 4, wobei die Polyole unterschiedlich sind von b).

Die Komponente f) wird bevorzugt in Mengen von ≥ 0 und ≤ 20 Gew.-%, besonders bevorzugt ≥ 0 und ≤ 10 Gew.-%, bezogen auf das Gesamtgewicht des Polyurethanharnstoffs, und ganz besonders bevorzugt gar nicht eingesetzt.

Bevorzugt weisen die Polyole der Komponente f) ein zahlenmittleres Molekulargewicht Mn ≥ 1000 und ≤ 3000 g/mol und einer Hydroxylfunktionalität von ≥ 1,8 und ≤ 3.

Als Komponente f) geeignete Polyole sind die in der Polyurethanlacktechnologie an sich bekannten Polyesterpolyole, Polyacrylatpolyole, Polyurethanpolyole, Polycarbonatpolyole, Polyesterpolyacrylatpolyole, Polyurethanpolyacrylatpolyole, Polyurethanpolyesterpolyole, Polyurethanpolyetherpolyole, Polyurethanpolycarbonatpolyole, Polyetherpolycarbonatpolyole und/oder Polyesterpolycarbonatpolyole, insbesondere Polyesterpolyole und/oder Polycarbonatpolyole.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Hydroxylfunktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure, ganz besonders bevorzugt ist Adipinsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

In der Komponente f) können auch Hydroxylgruppen aufweisende Polycarbonate, bevorzugt Polycarbonatdiole, mit zahlenmittleren Molekulargewichten Mn von 400 bis 8000 g/mol, bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art. Die Hydroxylgruppen aufweisenden Polycarbonate sind bevorzugt linear gebaut.

In einer bevorzugten Ausführungsform der Erfindung ist der erfindungsgemäß verwendete Polyurethanharnstoff aufgebaut aus
a) wenigstens einem aliphatischen, araliphatischen und/oder cycloaliphatischen Diisocyanat, das mindestens eine Isocyanatgruppe aufweist, die an ein sekundäres und/oder tertiäres C-Atom gebunden ist,
b) wenigstens einem Polyetherpolyol mit einem zahlenmittleren Molekulargewicht Mn ≥ 500 und ≤ 2500 g/mol und einer Hydroxylfunktionalität von ≥ 1,9 und ≤ 3,
c) wenigstens einer aminofunktionellen Verbindung, die mindestens zwei (isocyanatreaktive) Aminogruppen aufweist und ausgewählt ist aus Ethylendiamin, IPDA und/oder H12-MDA,
d) gegebenenfalls wenigstens ein Alkohol, der mindestens zwei Hydroxylgruppen und eine Molmasse ≥ 60 und ≤ 399 g/mol aufweist,
e) gegebenenfalls wenigstens eine Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Weiterhin bevorzugt ist der Polyurethanharnstoff in dieser vorgenannten Ausführungsform aufgebaut aus ≥ 5 und ≤ 60 Gew.-% der Komponente a), ≥ 30 und ≤ 90 Gew.-% der Komponente b), ≥ 2 und ≤ 25 Gew.-% der Komponente c), ≥ 0 und ≤ 10 Gew.-% der Komponente d), ≥ 0 und ≤ 10 Gew.-% der Komponente e) und ≥ 0 und ≤ 20 Gew.-% der Komponente f), jeweils bezogen auf die Gesamtmasse des Polyurethanharnstoffs, wobei sich die Komponenten a) bis f) zu 100 Gew.-% ergänzen.

Besonders bevorzugt ist der Polyurethanharnstoff in dieser vorgenannten Ausführungsform aufgebaut aus ≥ 10 und ≤ 40 Gew.-% der Komponente a), ≥ 55 und ≤ 85 Gew.-% der Komponente b), ≥ 5 und ≤ 20 Gew.-% der Komponente c), ≥ 0 und ≤ 3 Gew.-% der Komponente d), ≥ 0 und ≤ 3 Gew.-% der Komponente e) und ≥ 0 und ≤ 1 Gew.-% der Komponente f), jeweils bezogen auf die Gesamtmasse des Polyurethanharnstoffs, wobei sich die Komponenten a) bis f) zu 100 Gew.-% ergänzen.

In einer besonders bevorzugten Ausführungsform der Erfindung ist der erfindungsgemäß verwendete Polyurethanharnstoff aufgebaut aus
a) wenigstens einem Diisocyanat ausgewählt aus IPDI und/oder H12-MDI,
b) wenigstens einem Polyetherpolyol mit einem zahlenmittleren Molekulargewicht Mn ≥ 500 und ≤ 2500 g/mol und einer Hydroxylfunktionalität von ≥ 1,9 und ≤ 3, ausgewählt aus Polypropylenglykolen und/oder Poly(tetramethylenglykol)polyetherpolyolen,
c) wenigstens einer aminofunktionellen Verbindung, ausgewählt aus IPDA und/oder H12-MDA,
d) gegebenenfalls wenigstens ein Alkohol, der mindestens zwei Hydroxylgruppen und eine Molmasse ≥ 60 und ≤ 399 g/mol aufweist,
e) gegebenenfalls wenigstens eine Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Weiterhin bevorzugt ist der Polyurethanharnstoff in dieser vorgenannten Ausführungsform aufgebaut aus ≥ 5 und ≤ 60 Gew.-% der Komponente a), ≥ 30 und ≤ 90 Gew.-% der Komponente b), ≥ 2 und ≤ 25 Gew.-% der Komponente c), ≥ 0 und ≤ 10 Gew.-% der Komponente d), ≥ 0 und ≤ 10 Gew.-% der Komponente e) und ≥ 0 und ≤ 20 Gew.-% der Komponente f), jeweils bezogen auf die Gesamtmasse des Polyurethanharnstoffs, wobei sich die Komponenten a) bis f) zu 100 Gew.-% ergänzen.

Besonders bevorzugt ist der Polyurethanharnstoff in dieser vorgenannten Ausführungsform aufgebaut aus ≥ 10 und ≤ 40 Gew.-% der Komponente a), ≥ 55 und ≤ 85 Gew.-% der Komponente b), ≥ 5 und ≤ 20 Gew.-% der Komponente c), ≥ 0 und ≤ 3 Gew.-% der Komponente d), ≥ 0 und ≤ 3 Gew.-% der Komponente e) und ≥ 0 und ≤ 1 Gew.-% der Komponente f), jeweils bezogen auf die Gesamtmasse des Polyurethanharnstoffs, wobei sich die Komponenten a) bis f) zu 100 Gew.-% ergänzen.

Bevorzugt ist der Polyurethanharnstoff ausschließlich aufgebaut aus den Komponenten a) bis c) und gegebenenfalls d) bis f), besonders bevorzugt ausschließlich aus den Komponenten a) bis c).

Vorteilhaft weist der Polyurethanharnstoff ein zahlenmittleres Molekulargewicht Mn ≥ 2000 und ≤ 50000 g/mol, besonders vorteilhaft ≥ 3000 und ≤ 30000 g/mol, auf.

Der Polyurethanharnstoff wird bevorzugt hergestellt, indem die Komponenten a) und b) sowie gegebenenfalls d) und f) in einem ersten Schritt zu einem NCO-terminierten Präpolymer umgesetzt werden, welches dann in einem darauf folgenden Schritt mit der Komponente c) sowie gegebenenfalls den Komponenten d) und e) umgesetzt wird.

Für die Herstellung der Polyurethanharnstoffe werden bevorzugt die Komponenten a) und b) sowie gegebenenfalls d) und f) zur Herstellung eines NCO-terminierten Präpolymers ganz oder teilweise vorgelegt, gegebenenfalls mit einem gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden. In einer bevorzugten Variante wird jedoch ohne den Zusatz von Urethanisierungs-Katalysatoren gearbeitet.

Anschließend werden die gegebenenfalls zu Beginn der Reaktion noch nicht zugegebenen Bestandteile von a) und b) sowie gegebenenfalls d) und f) zudosiert.

Bei der Herstellung der NCO-terminierten Präpolymere aus den Komponenten a) und b) sowie gegebenenfalls d) und f) beträgt das Stoffmengenverhältnis von Isocyanat-Gruppen zu isocyanatreaktiven Gruppen im allgemeinen ≥ 1,05 und ≤ 3,5, bevorzugt ≥ 1,1 und ≤ 3,0, besonders bevorzugt ≥ 1,1 und ≤ 2,5.

Unter isocyanatreaktiven Gruppen sind alle gegenüber Isocyanat-Gruppen reaktiven Gruppen zu verstehen, wie beispielsweise primäre und sekundäre Aminogruppen, Hydroxygruppen oder Thiolgruppen.

Die Umsetzung der Komponenten a) und b) sowie gegebenenfalls d) und f) zum Präpolymer erfolgt teilweise oder vollständig, bevorzugt aber vollständig. Es werden so Polyurethan-Präpolymere, die freie Isocyanatgruppen enthalten, in Substanz oder in Lösung erhalten.

Bevorzugt wir das NCO-terminierte Präpolymer ausschließlich aus den Komponenten a) und b) hergestellt.

Im Anschluss wird bevorzugt in einem weiteren Verfahrensschritt, falls noch nicht oder nur teilweise geschehen, das erhaltene Präpolymer mit Hilfe von einem oder mehreren organischen Lösemitteln gelöst. Als Lösemittel wird dabei bevorzugt ebenfalls ein Lösemittel oder Lösemittelgemisch eingesetzt, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird. Für das Lösemittel und das Lösemittelgemisch gelten ebenfalls die nachstehenden bevorzugten Ausführungsformen zum Lösemittel oder Lösemittelgemisch in dem der Polyurethanharnstoff gelöst ist. Das Lösemittel oder Lösemittelgemisch kann dabei auch von dem Lösemittel oder Lösemittelgemisch, in dem der Polyurethanharnstoff als Endprodukt später gelöst ist, verschieden sein. Bevorzugt ist das Lösemittel oder Lösemittelgemisch identisch mit dem Lösemittel oder Lösemittelgemisch, in dem der Polyurethanharnstoff als Endprodukt später gelöst ist.

Bevorzugt besteht das bei der Herstellung eingesetzte Lösemittel aus einem oder mehreren monohydroxyfunktionellen Alkoholen.

Das Verhältnis von Lösemittel zu Präpolymer liegt dabei bevorzugt bei ≥ 1:10 und ≤ 5:1, besonders bevorzugt bei ≥ 1:2 und ≤ 2:1 Gewichtsteilen.

Das Präpolymer wird bevorzugt vor dem Lösen abgekühlt auf Temperaturen von -20 bis 60°C, vorzugsweise 0 bis 50°C und besonders bevorzugt von 15 bis 40°C.

In einem, sich gegebenenfalls an das Lösen des NCO-terminierten Präpolymers anschließenden, weiteren Schritt wird bevorzugt dann das im ersten Schritt erhaltene NCO-terminierte Präpolymer ganz oder teilweise mit der Komponente c) sowie gegebenenfalls den Komponenten d) und e) umgesetzt. Diese Umsetzung wird im allgemeinen als Kettenverlängerung, bzw. im Fall der Komponente e) als Kettenabbruch bezeichnet.

Bevorzugt wird dabei das NCO-terminierte Präpolymer vorgelegt und die Komponenten c) sowie gegebenenfalls d) und e) zudosiert. Bevorzugt erfolgt zunächst eine teilweise Umsetzung der NCO-Gruppen des Präpolymers mit den Komponenten c) und gegebenenfalls d) und im Anschluss der Kettenabbruch durch Umsetzung der verbleibenden NCO Gruppen mit der Komponente e). Die Komponenten c) und gegebenenfalls e) können dabei auch stufenweise in mehreren Schritten, insbesondere in zwei Schritten, zugegeben werden.

Die Komponente c) sowie gegebenenfalls d) und e) werden bevorzugt gelöst in einem oder mehreren organischen Lösemitteln eingesetzt. Als Lösemittel wird dabei bevorzugt ebenfalls ein Lösemittel oder Lösemittelgemisch eingesetzt, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird. Für das Lösemittel und das Lösemittelgemisch gelten ebenfalls die nachstehenden bevorzugten Ausführungsformen zum Lösemittel oder Lösemittelgemisch in dem der Polyurethanharnstoff gelöst ist. Das Lösemittel oder Lösemittelgemisch kann dabei auch von dem Lösemittel oder Lösemittelgemisch, in dem der Polyurethanharnstoff als Endprodukt später gelöst ist, verschieden sein. Bevorzugt ist das Lösemittel oder Lösemittelgemisch identisch mit dem Lösemittel oder Lösemittelgemisch, in dem der Polyurethanharnstoff als Endprodukt später gelöst ist.

Bevorzugt besteht das bei der Herstellung eingesetzte Lösemittel für Komponente c) aus einem oder mehreren monohydroxyfunktionellen Alkoholen.

Wenn Lösemittel als Verdünnungsmittel verwendet werden, so beträgt der Verdünnungsmittelgehalt in der bei der Kettenverlängerung eingesetzten Komponenten c), sowie gegebenenfalls d) und e) bevorzugt 1 bis 95 Gew.-%, besonders bevorzugt 3 bis 50 Gew.-%, bezogen auf das Gesamtgewicht der Komponente c) sowie gegebenenfalls d) und e) einschließlich Verdünnungsmittel.

Die Zugabe der Komponenten c) sowie gegebenenfalls d) und e) erfolgt bevorzugt bei Temperaturen von -20 bis 60°C, vorzugsweise 0 bis 50 und besonders bevorzugt von 15 bis 40°C.

Der Kettenverlängerungsgrad, also das molare Verhältnis von NCO-reaktiven Gruppen der zur Kettenverlängerung und Kettenabbruch eingesetzten Komponenten c) sowie gegebenenfalls d) und e) zu freien NCO-Gruppen des Präpolymers, liegt im allgemeinen ≥ 50 und ≤ 150%, bevorzugt ≥ 50 und ≤ 120%, besonders bevorzugt ≥ 60 und ≤ 100% und ganz besonders bevorzugt ≥ 70 und ≤ 95%.

Bevorzugt beträgt das molare Verhältnis der isocyanatreaktiven Gruppen der Komponente c) zu den freien NCO-Gruppen des Präpolymers ≥50 und ≤ 120%, besonders bevorzugt ≥ 60 und ≤ 100% und ganz besonders bevorzugt ≥ 70 und ≤ 95%.

In einer bevorzugten Ausführungsform der Erfindung werden die freien NCO-gruppen des Präpolymers nur teilweise mit der Komponente c) umgesetzt, bevorzugt beträgt dabei das molare Verhältnis der isocyanatreaktiven Gruppen der Komponente c) zu den freien NCO-Gruppen des Präpolymers ≥ 60 und ≤ 95%, und die verbleibenden freien NCO Gruppen reagieren mit den Hydroxy-gruppen des Lösemittels ab, so dass ein NCO freier Polyurethanharnstoff entsteht.

Im Anschluss an die Herstellung kann der Polyurethanharnstoff, sofern im Herstellungsverfahren bereits erfindungsgemäße Lösemittel oder Lösemittelgemische eingesetzt wurden, weiterhin mit einem Lösemittel oder Lösemittelgemisch verdünnt und dabei gelöst werden, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunk-tionellen Alkohol enthält, verwendet wird.

Wurden während der Umsetzung keine erfindungsgemäßen Lösemittel oder Lösemittelgemische eingesetzt, so wird im Anschluss an die Herstellung des Polyurethanharnstoffs dieser in einem Lösemittel oder Lösemittelgemisch eingesetzt, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird.

Das Lösen des Polyurethanharnstoffs kann mit den üblichen Techniken zur Scherung erfolgen, beispielsweise durch Rühren mit Standard-Rührern, beispielsweise wie in DIN 28131 genannt.

Das Lösen des Polyurethanharnstoffs erfolgt vorzugsweise ohne die zusätzliche Zugabe von externen Emulgatoren. Die erfindungsgemäße verwendeten Polyurethanharnstofflösungen umfassen vorzugsweise keine externen Emulgatoren.Als Lösemittel oder Bestandteil des Lösemittelgemisches geeignet sind prinzipiell alle monohydroxyfunktionellen, aliphatischen Alkohole mit einem bis sechs Kohlenstoffatomen, wie beispielsweise Methanol, Ethanol, n-Propanol, iso-Propanol, n-Butanol, sec.-Butanol und/oder Butylglykol. Besonders bevorzugt handelt es sich bei dem monohydroxyfunktionellen Alkohol um Ethanol.

Wird ein Lösemittelgemisch eingesetzt, so können neben den monohydroxyfunktionellen Alkoholen noch ≤ 50 Gew.-%, bezogen auf die Gesamtmasse des Lösemittelgemisches, eines weiteren organischen Lösemittels eingesetzt werden. Geeignete Lösemittel sind dabei sind beispielsweise Ester, wie z.B. Ethylacetat, Butylacetat, Methyoxypropylacetat oder Butyrolacton, Ketone, wie z.B. Aceton oder Methylethylketon, Ether, wie z.B. Tetrahydrofuran oder tert.-Butylmethylether, aromatische Lösungsmittel, wie z.B. Xylol oder Solvent Naphtha. Im Falle der Verwendung von Ethanol können typische Vergällungsmittel als Additive in den üblichen Zuschlagmengen enthalten sein.

Bevorzugt beträgt der Anteil der weiteren organischen Lösemittel ≤ 30 Gew.-%, besonders bevorzugt ≤ 5 Gew.-% und ganz besonders bevorzugt ≤ 2 Gew.-%, bezogen auf das Gesamtgewicht des Lösemittelgemisches,. In einer ganz besonders bevorzugten Ausführungsform sind neben monohydroxyfunktionellen, aliphatischen Alkoholen keinerlei weitere organische Lösemittel enthalten.

Als weitere Lösemittel ungeeignet sind physiologisch unverträgliche Lösemittel wie beispielsweise Dimethylformamid, N-Methylpyrrolidon oder Toluol, wie sie oft als Colösemittel für Polyurethane oder Polyurethanharnstoffe eingesetzt werden, das diese vorzugsweise nicht in kosmetischen Zusammensetzungen enthalten sein sollten.

Bei den weiteren Lösemitteln handelt es sich auch nicht um Wasser. Die durch Lösen des Polyurethanharnstoffs in den erfindungsgemäß verwendeten Lösemitteln oder Lösemittelgemischen erhaltene Polyurethanharnstofflösung ist bevorzugt wasserfrei, ausgenommen der Anteile an Wasser, die in den eingesetzten organischen Lösemitteln herstellungsbedingt enthalten sind.

Der Wasseranteil der Polyurethanharnstofflösung liegt bevorzugt bei ≤ 10 Gew.-%, besonders bevorzugt bei ≤ 4,5 Gew.-% und ganz besonders bevorzugt bei ≤ 1 Gew.-%, bezogen auf die Gesamtmasse der Polyurethanharnstofflösung.

Der Anteil des Polyurethanharnstoffs (als Aktivstoff) an der erfindungsgemäß eingesetzten Polyurethanharnstofflösung (auch bezeichnet als Feststoffgehalt) liegt dabei bevorzugt ≥ 10 und ≤ 80 Gew.-%, besonders bevorzugt ≥ 15 und ≤ 60 Gew.-% und ganz besonders bevorzugt bei ≥ 20 und ≤ 50 Gew.-%, bezogen auf das Gesamtgewicht der Polyurethanharnstofflösung.

Das erfindungsgemäße Verfahren eignet sich zur Herstellung von kosmetischen Zusammensetzungen bzw. die durch das erfindungsgemäße Verfahren erhältlichen kosmetischen Zusammensetzungen sind bevorzugt solche, die zur Behandlung von Nägeln, der Haut und/oder von keratinen Fasern, bevorzugt von Haut und/oder Haaren, besonders bevorzugt der Haut, angewendet werden, insbesondere handelt es sich um Sonnenschutz-Zusammensetzungen. Besonders bevorzugt handelt es sich um Sonnenschutz-Zusammensetzungen zur Anwendung auf der Haut.

Unter kosmetischen Zusammensetzungen zur Behandlung von Nägeln sind insbesondere Nagellacke zu verstehen.

Unter Nägeln sind im Sinne dieser Erfindung Finger- und/oder Fußnägel zu verstehen.

Ein weiterer Gegenstand der Erfindung ist eine kosmetische Zusammensetzung erhältlich nach dem erfindungsgemäßen Verfahren.

Das erfindungsgemäße Verfahren eignet sich zur Herstellung von kosmetischen Zusammensetzungen bzw. die durch das erfindungsgemäße Verfahren erhältlichen kosmetischen Zusammensetzungen sind bevorzugt solche, die in Form von Gelen, Ölen, Sprays und Aerosolen vorliegen, die vorzugsweise transparent sind. Transparent im Sinne dieser Erfindung bedeutet, dass die Trübungswerte der Zusammensetzung ≤ 100 NTU (Nephelometric Turbidity Unit), bevorzugt ≤ 50 NTU, besonders bevorzugt ≤ 10 NTU und ganz besonders bevorzugt ≤ 5 NTU sind. Die Trübungswerte werden dabei durch eine Streulichtmessung im 90°-Winkel (Nephelometrie) bei einer Wellenlänge der Mess-Strahlung 860 nm entsprechend DIN EN ISO 7027 bestimmt, durchgeführt bei 23°C mit einem Labortrübungsmessgerät Modell 2100AN der Firma HACH LANGE GmbH, Berlin, Deutschland.

Bevorzugt handelt es sich bei den kosmetischen Zusammensetzungen um solche die überwiegend Alkohol basiert sind, also ≥ 10 und ≤ 90 Gew.-%, bezogen auf die Gesamtmasse der kosmetischen Zusammensetzung, bevorzugt ≥ 15 und ≤ 70 Gew.-% und besonders bevorzugt ≥ 20 und ≤ 60 Gew.-% an aliphatischen Alkoholen, mit 1 bis 6 Kohlenstoffatomen, bevorzugt 1 bis 4 Kohlenstoffatomen enthalten. Die Alkohole sind dabei bevorzugt ausgewählt aus Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, oder deren Gemischen. Besonders bevorzugt enthalten die Alkohole Ethanol oder bestehen daraus, ganz besonders bevorzugt handelt es sich bei dem verwendeten Alkohol um Ethanol.

Besonders bevorzugt handelt es sich bei den kosmetischen Zusammensetzungen um alkoholische Lösungen.

Die kosmetischen Zusammensetzungen enthalten einen Wasseranteil von ≥ 0 und ≤ 5 Gew.-% und weiterhin bevorzugt ≥ 0 und < 2 Gew.-%. Insbesondere bevorzugt sind die kosmetischen Zusammensetzungen wasserfrei, enthalten somit nicht mehr Wasser, als das was herstellungsbedingt über die Rohstoffe unvermeidbar in die Formulierung eingebracht wird.

Der Anteil der in der kosmetischen Zusammensetzung eingesetzten Polyurethanharnstofflösung beträgt bevorzugt ≥ 0,5 und ≤ 80 Gew.-%, besonders bevorzugt ≥ 1 und ≤ 60 Gew.-% und ganz besonders bevorzugt ≥ 2 und ≤ 40 Gew.-%, bezogen auf die Gesamtmasse der kosmetischen Zusammensetzung.

Der Feststoffgehalt der Polyurethanharnstofflösung ist dabei bevorzugt so gewählt, dass die kosmetischen Zusammensetzungen bevorzugt ≥ 0,1 Gew.-% und ≤ 30 Gew.-%, besonders bevorzugt ≥ 0,5 und ≤ 20 Gew.-% und ganz besonders bevorzugt ≥ 1 und ≤ 10 Gew.-% des Polyurethanharnstoffs als Aktivstoff, bezogen auf die Gesamtmasse der kosmetischen Zusammensetzung, enthalten.

Als Aktivstoff wird der Polyurethanharnstoff ohne Lösemittel oder Lösemittelgemisch verstanden.

Die kosmetischen Zusammensetzungen enthalten zudem bevorzugt in der Kosmetik übliche Additive wie Emulgatoren, grenzflächenaktive Stoffe, Entschäumer, Verdicker, Tenside, Feuchthaltemittel, Füllstoff, Filmbildner, Lösemittel, Koaleszenzmittel, Gelbildner und/oder andere Polymerdispersionen wie beispielsweise Dispersionen auf Basis von Polyacrylaten, Füllstoffe, Weichmacher, Pigmente, Farbstoffe, Verlaufsmittel, Thixotropiemittel, Geschmeidigkeitsmittel, Konservierungsmittel, sensorische Additive, Öle, Wachse und/oder Treibgase wie zum Beispiel Propan/Butan oder Dimethyl Ether etc.. Die Mengen der verschiedenen Additive sind dem Fachmann für den einzusetzenden Bereich bekannt und liegen beispielweise im Bereich von ≥ 0 und ≤ 40 Gew.-%, bevorzugt ≥ 0,1 und ≤ 40 Gew. -% bezogen auf das Gesamtgewicht der kosmetischen Zusammensetzung.

Bevorzugt enthalten die kosmetischen Zusammensetzungen auch Sonnenschutzfiltersubstanzen, insbesondere UV-Absorber. Der Anteil der Sonnenschutzfiltersubstanzen an der Gesamtmasse der kosmetischen Zusammensetzung beträgt bevorzugt ≥ 0,01 und ≤ 40 Gew.-%, besonders bevorzugt ≥ 1 und ≤ 35 Gew.-% und ganz besonders bevorzugt ≥ 5 und ≤ 30 Gew.-% .

Die Begriffe Sonnenschutzfilter-Substanzen und UV-Filter-Substanzen werden im Rahmen dieser Anmeldung als äquivalente Begriffe verwendet.

Die Sonnenschutzfiltersubstanzen (oder UV-Filter) können unter den organischen Filtern, den physikalischen Filtern und/oder deren Gemischen ausgewählt werden, bevorzugt sind jedoch organische Filter, insbesondere bevorzugt öllösliche organische Filter. Geeignet sind an sich alle im Anhang VII der EU-Kosmetikverordnung (76/768/EEC) aufgeführten Sonnenschutzfiltersubstanzen.

Die eingesetzten UV-Filter können öllöslich und/oder wasserlöslich sein, wobei öllösliche UV-Filter bevorzugt sind. Bevorzugt enthält die kosmetische Zusammensetzung wenigstens einen öllöslichen UV-Filter.

Öllösliche UV-Filter können solche sein, die flüssig sind , insbesondere ölartig und selbst auch als Lösemittel für andere öllösliche UV-Filter dienen können oder solche die fest sind und in Ölen gelöst eingesetzt werden.

Bevorzugt eingesetzte, flüssige, öllösliche UV-Filter sind Octocrylene, Ethylhexylmethoxycinnamate, Ethylhexylsalicylate und/oder Homosalate.

Bevorzugt eingesetzte, feste, öllösliche UV-Filter sind Butylmethoxydibenzoylmethan (Avobenzone), Dioctylbutylamidotriazon (INCI: Diethylhexyl Butamidotriazone), 2 ,4-B is-{[4-(2-ethyl-he-xyloxy)-2-hyd roxy]-ph enyl}-6-(4-methoxyphenyl)-1,3,5-triazin (INCI: Bis-Ethylhexyloxyphenol Methoxyphenyl Triazin), Ethylhexyl Triazon, Diethylamino Hydroxybenzoyl Hexy 1Benzoat, Benzophenone-3. Darüber hinaus können auch alle anderen öllöslichen Filter die im Anhang VII der EU-Kosmetikverordnung (76/768/EEC) aufgeführt sind eingesetzt werden.

In einer bevorzugten Ausführungsform der Erfindung enthalten die kosmetischen Zusammensetzungen wenigstens einen flüssigen, öllöslichen UV-Filter.

Die kosmetischen-Zusammensetzungen enthaltend UV-Filter umfassen auch solche kosmetischen Zusammensetzungen, deren hauptsachlicher Zweck nicht der Schutz vor Sonnenlicht ist, die aber dennoch einen Gehalt an UV-Filtern enthalten. So werden beispielsweise in Tagescremes oder Makeup-Produkten gewöhnlich UV-A- bzw. UV-B-Filtersubstanzen eingearbeitet. Auch Haarpflegeprodukt oder Nagellacke können UV-Filtersubstanzen enthalten. Darüber hinaus stellen UV-Schutzsubstanzen, ebenso wie Antioxidantien und Konservierungsstoffe, einen wirksamen Schutz der Zubereitungen selbst gegen Verderb dar.

Die kosmetischen Zusammensetzungen können Öle und/oder Wachse enthalten, wobei es sich bei den Ölen um nicht flüchtige und/oder flüchtige Öle handeln kann.

Die kosmetische Zusammensetzung enthält vorteilhaft ≥ 0 und ≤ 45 Gew.-% Öle, bezogen auf das Gesamtgewicht der Zusammensetzung, und besonders vorteilhaft ≥ 0,01 und ≤ 20 Gew:-% Öle. Die Wachse können in Mengen von ≥ 0 und ≤ 10 Gew.-%, bezogen auf das Gesamtgewicht der Zusammensetzung und vorzugsweise ≥ 1 und ≤ 5 Gew.-% enthalten sein.

In einer bevorzugten Ausführungsform der Erfindung enthält die kosmetische Zusammensetzung
A) wenigstens einen Polyurethanharnstoff, welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird,
B) wenigstens eine öllösliche Sonnenschutzfilter-Substanz und
C) wenigstens einen aliphatischen Alkohol mit 1 bis 6 C-Atomen.

In einer besonders bevorzugten Ausführungsform der Erfindung enthält die kosmetische Zusammensetzung
A) ≥ 0,5 und ≤ 80 Gew.-% wenigstens eines Polyurethanharnstoff, welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird,
B) ≥ 0,01 und ≤ 40 Gew.-% an Sonnenschutzfilter-Substanzen, enthaltend wenigstens eine öllöslichen Sonnenschutzfilter-Substanz,
C) ≥ 10 und ≤ 90 Gew.-% wenigstens eines aliphatischen Alkohols mit 1 bis 4 C-Atomen und
D) ≥ 0 und ≤ 45 Gew.-% wenigstens eines Öl und/oder ≥ 0 und ≤ 10 Gew.-% wenigstens eines Wachses,
E) ≥ 0 und ≤ 40 Gew.- % in der Kosmetik übliche Additive,
jeweils bezogen auf die Gesamtmasse der kosmetischen Zusammensetzung, wobei die Komponenten A) bis D) sich zu 100 Gew.-% ergänzen.

Dabei ist die Komponente D) bevorzugt nur dann = 0 wenn die Komponente B) bereits wenigstens eine flüssige, öllösliche Sonnenschutzfilter-Substanz enthält. Diese kann dann als Öl angesehen werden, das auch weitere feste öllösliche Sonnenschutz-Filtersubstanzen lösen kann.

Der Feststoffgehalt der Polyurethanharnstofflösung ist dabei bevorzugt so gewählt, dass die kosmetischen Zusammensetzungen bevorzugt ≥ 0,5 und ≤ 20 Gew.-% des Polyurethanharnstoffs als Aktivstoff, bezogen auf die Gesamtmasse der kosmetischen Zusammensetzung, enthalten.

Das erfindungsgemäße Verfahren umfasst bevorzugt einen Schritt in dem eine homogene Ölphase aus den öllöslichen UV-Filtersubstanzen mit gegebenenfalls weiteren Ölen und Wachsen, sowie gegebenenfalls weiteren Additiven hergestellt wird. Dieser Schritt wird bevorzugt bei erhöhten Temperaturen, besonders bevorzugt ≥ 20 °C und ≤ 90 °C durchgeführt. Wird die Ölphase bei Temperaturen oberhalb der Raumtemperatur hergestellt, kann diese im Anschluss an die Herstellung abgekühlt werden, bevorzugt auf Raumtemperatur.

Weiterhin umfasst das erfindungsgemäße Verfahren bevorzugt einen Schritt, bei dem die Ölphase mit einer zweiten Phase vermischt wird, die wenigstens die Polyurethanharnstofflösung enthält. Bevorzugt enthält diese weitere Phase außerdem wenigstens einen aliphatischen Alkohol mit 1 bis 6, bevorzugt 1 bis 4 C-Atomen.

Additive können zu jedem Zeitpunkt im Verfahren in die kosmetische Zusammensetzung eingebracht werden.

Ein weiterer Gegenstand der Erfindung ist ein Verfahren zur Herstellung einer kosmetischen Beschichtung auf Haut, Nägel und/oder keratine Fasern, bevorzugt auf Haut und/oder Haare, besonders bevorzugt auf die Haut unter Verwendung der erfindungsgemäßen kosmetischen Zusammensetzungen, wobei die kosmetische Zusammensetzung auf Haut, Nägel und/oder keratine Fasern, bevorzugt auf Haut und/oder Haare, besonders bevorzugt auf die Haut aufgetragen wird.

Vorteilhaft verbleiben bei dem erfindungsgemäßen Verfahren die erfindungsgemäßen kosmetischen Zusammensetzungen zumindest teilweise auf Haut, Nägel und/oder keratine Fasern, bevorzugt auf Haut und/oder Haare, besonders bevorzugt auf der Haut.

Ein weitere Gegenstand der Erfindung ist einen Sonnenschutz-Zusammensetzung enthaltend wenigstens eine öllösliche organische Sonnenschutzfiltersubstanz und wenigstens einen Polyurethanharnstoff, welcher keine ionisch hydrophilierenden Gruppen aufweist und welcher gelöst in einem Lösemittel oder Lösemittelgemisch eingesetzt wird, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird. Der verwendete Polyurethanharnstoff ist dabei aufgebaut aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Die Polyurethanharnstoffe der vorliegenden Erfindung werden zur Herstellung der erfindungsgemäßen Sonnenschutz-Zusammensetzung gelöst in einem Lösemittel oder Lösemittelgemisch, und somit als Polyurethanharnstofflösungen und nicht als wässrige Dispersion, eingesetzt.

Ionisch hydrophilierende Gruppen im Sinne der Erfindung sind solche die in den Polyurethanharnstoff beispielsweise durch geeignete anionisch bzw. potentiell anionisch hydrophilierenden Verbindungen eingebracht werden könnten. Diese weisen mindestens eine isocyanatreaktive Gruppe, wie eine Hydroxylgruppe oder Aminogruppe auf, sowie mindestens eine Funktionalität wie z.B. -COO-M⁺, -SO₃-M⁺, -PO(O-M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5 - 9, Formel I-III beschrieben ist.

Unter potentiell anionischen (und im Allgemeinen potentiell ionischen) Gruppen sind im chemische Reaktion, insbesondere durch Neutralisation in eine anionische (ionische) Gruppe überführt werden können.

In einer bevorzugten Ausführungsform des erfindungsgemäßen Verfahrens weist der eingesetzte Polyurethanharnstoff keine hydrophilierenden Gruppen, also weder ionisch, noch nicht-ionisch hydrophilierende Gruppen auf.

Nicht-ionisch hydrophilierende Gruppen im Sinne der Erfindung sind solche, die in den Polyurethanharnstoff beispielsweise durch geeignete nicht-ionisch hydrophilierende Verbindungen eingeführt werden könnten, wie beispielsweise Polyoxyalkylenether, die mindestens eine Hydroxy- oder Aminogruppe enthalten. Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38 beschrieben). Diese Verbindungen sind entweder reine Polyethylenoxidether oder gemischte Polyalkylenoxidether, wobei sie dann aber mindestens 30 mol-%, bevorzugt mindestens 40 mol-%, bezogen auf alle enthaltenen Alkylenoxideinheiten an Ethylenoxideinheiten, enthalten.

### Der erfindungsgemäß verwendete Polyurethanharnstoff aufgebaut aus

a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Weiterhin bevorzugt ist der Polyurethanharnstoff aufgebaut aus ≥ 5 und ≤ 60 Gew.-% der Komponente a), ≥ 30 und ≤ 90 Gew-% der Komponente b), ≥ 2 und ≤ 25 Gew.-% der Komponente c), ≥ 0 und ≤ 10 Gew.-% der Komponente d), ≥ 0 und ≤ 10 Gew.-% der Komponente e) und ≥ 0 und ≤ 20 Gew.-% der Komponente f), jeweils bezogen auf die Gesamtmasse des Polyurethanharnstoffs, wobei sich die Komponenten a) bis f) zu 100 Gew.-% ergänzen.

Besonders bevorzugt ist der Polyurethanharnstoff aufgebaut aus ≥ 10 und ≤ 40 Gew.-% der Komponente a), ≥ 55 und ≤ 85 Gew.-% der Komponente b), ≥ 5 und ≤ 20 Gew.-% der Komponente c), ≥ 0 und ≤ 3 Gew.-% der Komponente d), ≥ 0 und ≤ 3 Gew.-% der Komponente e) und ≥ 0 und ≤ 1 Gew.-% der Komponente f), jeweils bezogen auf die Gesamtmasse des Polyurethanharnstoffs, wobei sich die Komponenten a) bis f) zu 100 Gew.-% ergänzen.

Als Komponente a) geeignete Verbindungen sind beispielsweise 1,4-Butylendiisocyanat, 1,5-Pentamethy¬lendiisocyanat (PDI), 1,6-Hexamethy-lendiisocyanat (HDI), Isophorondiisocyanat (IPDI), 2,2,4- und/oder 2,4,4-Trimethyl-hexa¬methy¬len¬¬diiso¬cyanat, die isomeren Bis-(4,4'-isocyanatocyclo¬hexyl)¬methane oder deren Mischungen beliebigen Isomeren¬gehalts (H12-MDI), 1,4-Cyclohexylendi-isocyanat, 4-Isocyanatomethyl-1,8-octandiisocyanat (Nonantriisocyanat), 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin¬diisocyanate) mit C1-C8-Alkylgruppen.

Neben den vorstehend genannten Polyisocyanaten können anteilig auch modifizierte Diisocyanate oder Triisocyanate mit Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit einer mittleren NCO-Funktionalität von ≥ 2 und ≤ 4, bevorzugt von ≥ 2 und ≤ 2,6 und besonders bevorzugt von ≥ 2 und ≤ 2,4.

Bevorzugt ist die Komponente a) ausgewählt aus aliphatischen, araliphatischen und/oder cycloaliphatischen Diisocyanaten, welche mindestens eine Isocyanatgruppe aufweisen, die an ein sekundäres und/oder tertiäres C-Atom gebunden ist.

Besonders bevorzugt ist die Komponente a) ausgewählt aus IPDI und/oder H12-MDI.

Weiterhin bevorzugt werden zur Herstellung des Polyurethanharnstoffs keine aromatischen Polyisocyanate eingesetzt.

Die Komponente a) wird bevorzugt in Mengen von ≥ 5 und ≤ 60 Gew.-%, besonders bevorzugt ≥ 10 und ≤ 40 Gew.-% und ganz besonders bevorzugt von ≥ 15 und ≤ 35 Gew.-% bezogen auf das Gesamtgewicht des Polyurethanharnstoffs, eingesetzt.

Die Komponente b) besteht aus einem oder mehreren Polyetherpolyolen mit einem zahlenmittleren Molekulargewicht Mn ≥ 400 und ≤ 6000 g/mol und einer Hydroxylfunktionalität von ≥ 1,5 und ≤ 4, bevorzugt mit einem zahlenmittleren Molekulargewicht Mn ≥ 500 und ≤ 2500 g/mol und einer Hydroxylfunktionalität von ≥ 1,9 und ≤ 3 und besonders bevorzugt mit einem zahlenmittleren Molekulargewicht Mn ≥ 1000 und ≤ 2000 g/mol und einer Hydroxylfunktionalität von ≥ 1,9 und ≤ 2,1.

Geeignete Polyetherpolyole der Komponente b) sind beispielsweise die in der Polyurethanchemie an sich bekannten Poly(tetramethylenglykol)polyetherpolyole, wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Die Polyetherpolyole weisen dabei bevorzugt einen Anteil an Gruppen erhalten aus Ethylenoxid von < 50 Gew.-%, bevorzugt < 30 Gew.-% auf.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Bevorzugt ist die Komponente b) ausgewählt aus Polypropylenglykolen und/oder Poly(tetramethylenglykol)polyetherpolyolen, besonders bevorzugt ausgewählt aus Poly(tetramethylenglykol)polyetherpolyolen.

In einer bevorzugten Ausführungsform der Erfindung handelt es sich bei der Komponente b) um ein oder mehrere Poly(tetramethylenglykol)polyetherpolyole mit einem mit einem zahlenmittleren Molekulargewicht Mn ≥ 500 und ≤ 2500 g/mol und einer Hydroxylfunktionalität von ≥ 1,9 und ≤ 2,1.

In einer besonders bevorzugten Ausführungsform ist die Komponente b) ein Gemisch aus Poly(tetramethylenglykol)polyetherpolyolen I mit einem zahlenmittleren Molekulargewichte Mₙ von ≥ 400 und ≤ 1500 g/mol, besonders bevorzugt von ≥ 600 und ≤ 1200 g/mol, ganz besonders bevorzugt von 1000 g/mol und Poly(tetramethylenglykol)polyetherpolyolen II mit einem zahlenmittleren Molekulargewichte Mₙ von ≥ 1500 und ≤ 8000 g/mol, besonders bevorzugt von ≥ 1800 und ≤ 3000 g/mol, ganz besonders bevorzugt von 2000 g/mol.

Das Gewichtsverhältnis der Poly(tetramethylenglykol)polyetherpolyole I zu den Poly(tetramethylenglykol)polyetherpolyolen II liegt bevorzugt im Bereich von ≥ 0,1 und ≤ 10, besonders bevorzugt im Bereich von ≥ 0,2 und ≤ 8, ganz besonders bevorzugt im Bereich von ≥ 1 und ≤ 6.

Die Komponente b) wird bevorzugt in Mengen von ≥ 30 und ≤ 90 Gew.-%, besonders bevorzugt ≥ 50 und ≤ 85 Gew.-%, ganz besonders bevorzugt ≥ 55 und ≤ 75 Gew.-% bezogen auf das Gesamtgewicht des Polyurethanharnstoffs, eingesetzt.

Bei der Komponente c) handelt es sich um eine oder mehrere aminofunktionelle Verbindungen, die mindestens zwei isocyanatreaktive Aminogruppen aufweisen.

Als Komponente c) sind beispielsweise geeignet Di- oder Polyamine wie 1,2-Ethylendiamin, 1,2- und 1,3-Diaminopropan, 1,4-Diaminobutan, 1,6-Diaminohexan, 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4'-Diaminodicyclohexylmethan (H12-MDA), Isophorondiamin (IPDA) und/oder 1,2-Dimethylethylendiamin.

Bevorzugt ist die Komponente c) ausgewählt aus Ethylendiamin, IPDA und/oder H12-MDA, besonders bevorzugt aus Isophorondiamin und/oder H12-MDA und ganz besonders bevorzugt ist die Komponente c) H12-MDA.

Die Verbindungen der Komponente c) enthalten bevorzugt keine hydrophilierenden Gruppen, insbesondere keine ionisch oder potentiell ionisch hydrophilierenden Gruppen.

In einer besonders bevorzugten Ausführungsform der Erfindung ist die Komponente c) ausgewählt aus Aminen, die mindestens zwei isocyanatreaktive Aminogruppen aufweisen, die an primäre und/oder sekundäre C-Atome gebunden sind.

Weiterhin bevorzugt ist die Komponente c) ausgewählt aus symmetrisch aufgebauten Diaminen. Ganz besonders bevorzugt ist die Komponente c) ausgewählt aus symmetrischen Diaminen, die mindestens zwei Aminogruppen aufweisen, die an primäre und/oder sekundäre C-Atome gebunden sind, insbesondere bevorzugt ist die Komponente c) H12-MDA.

Die Komponente c) wird bevorzugt in Mengen von ≥ 2 und ≤ 25 Gew.-%, besonders bevorzugt ≥ 5 und ≤ 20 Gew.-% und ganz besonders bevorzugt ≥ 9 und ≤ 16 Gew.-% bezogen auf das Gesamtgewicht des Polyurethanharnstoffs, eingesetzt.

In einer bevorzugten Ausführungsform der Erfindung ist entweder die Komponente a) H12-MDI oder die Komponente c) H12-MDA oder die Komponente a) H12-MDI und die Komponente c) H12-MDA.

Gegebenenfalls wird der Polyurethanharnstoff zudem aufgebaut aus Komponente d), ein oder mehrere Alkohole, die mindestens zwei Hydroxylgruppen und eine Molmasse von ≥ 60 und ≤ 399 g/mol aufweisen, wie beispielsweise Polyole des genannten Molmassenbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit.

Die Komponente d) wird bevorzugt in Mengen von ≥ 0 und ≤ 10 Gew.-%, besonders bevorzugt ≥ 0 und ≤ 3 Gew.-%, bezogen auf das Gesamtgewicht des Polyurethanharnstoffs, und ganz besonders bevorzugt gar nicht eingesetzt.

Weiterhin kann der Polyurethanharnstoff aufgebaut sein aus Komponente e), einer oder mehreren Verbindungen, die eine gegenüber Isocyanatgruppen reaktive Gruppe aufweisen, insbesondere Verbindungen die eine Amino- oder Hydroxygruppe aufweisen. Geeignete Verbindungen der Komponente e) sind beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Methanol, Ethanol, iso-Propanol, n-Propanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Komponente e) umfasst bevorzugt keine monofunktionellen Polyetherpolyole, die einen Anteil an Gruppen erhalten aus Ethylenoxid von > 30 Gew.-%, bevorzugt > 50 Gew.-% aufweisen.

Der als Lösemittel für den Polyurethanharnstoff eingesetzte monohydroxyfunktionelle Alkohol kann ebenfalls als Aufbaukomponente e) für den Polyurethanharnstoff dienen.

Die Komponente e) wird, bevorzugt in Mengen, von ≥ 0 und ≤ 10 Gew.-%, besonders bevorzugt ≥ 0 und ≤ 3 Gew.-%, bezogen auf das Gesamtgewicht des Polyurethanharnstoffs, und ganz besonders bevorzugt gar nicht eingesetzt, wobei hier der als Lösemittel für den Polyurethanharnstoff eingesetzte monohydroxyfunktionelle Alkohol als Komponente e) unberücksichtigt bleibt.

Der monohydroxyfunktionelle Alkohol, welcher als Lösemittel für den Polyurethanharnstoff dient, macht bevorzugt ≥ 0 und ≤ 5 Gew.-%, besonders bevorzugt ≥ 0,01 und ≤ 3 Gew.-% und ganz besonders bevorzugt ≥ 0,01 und ≤ 2 Gew.-% der Gesamtmasse des Polyurethanharnstoffs aus.

Gegebenenfalls kann der Polyurethanharnstoff auch aufgebaut sein aus der Komponente f), einem Polyol oder mehreren Polyolen mit einem zahlenmittleren Molekulargewicht Mn ≥ 500 und ≤ 6000 g/mol und einer Hydroxylfunktionalität von ≥ 1,5 und ≤ 4, wobei die Polyole unterschiedlich sind von b).

Die Komponente f) wird bevorzugt in Mengen von ≥ 0 und ≤ 20 Gew.-%, besonders bevorzugt ≥ 0 und ≤ 10 Gew.-%, bezogen auf das Gesamtgewicht des Polyurethanharnstoffs, und ganz besonders bevorzugt gar nicht eingesetzt.

Bevorzugt weisen die Polyole der Komponente f) ein zahlenmittleres Molekulargewicht Mn ≥ 1000 und ≤ 3000 g/mol und einer Hydroxylfunktionalität von ≥ 1,8 und ≤ 3.

Als Komponente f) geeignete Polyole sind die in der Polyurethanlacktechnologie an sich bekannten Polyesterpolyole, Polyacrylatpolyole, Polyurethanpolyole, Polycarbonatpolyole, Polyesterpolyacrylatpolyole, Polyurethanpolyacrylatpolyole, Polyurethanpolyesterpolyole, Polyurethanpolyetherpolyole, Polyurethanpolycarbonatpolyole, Polyetherpolycarbonatpolyole und/oder Polyesterpolycarbonatpolyole, insbesondere Polyesterpolyole und/oder Polycarbonatpolyole.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Hydroxylfunktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure, ganz besonders bevorzugt ist Adipinsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

In der Komponente f) können auch Hydroxylgruppen aufweisende Polycarbonate, bevorzugt Polycarbonatdiole, mit zahlenmittleren Molekulargewichten Mn von 400 bis 8000 g/mol, bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art. Die Hydroxylgruppen aufweisenden Polycarbonate sind bevorzugt linear gebaut.

In einer bevorzugten Ausführungsform der Erfindung ist der erfindungsgemäß verwendete Polyurethanharnstoff aufgebaut aus
a) wenigstens einem aliphatischen, araliphatischen und/oder cycloaliphatischen Diisocyanat, das mindestens eine Isocyanatgruppe aufweist, die an ein sekundäres und/oder tertiäres C-Atom gebunden ist,
b) wenigstens einem Polyetherpolyol mit einem zahlenmittleren Molekulargewicht Mn ≥ 500 und ≤ 2500 g/mol und einer Hydroxylfunktionalität von ≥ 1,9 und ≤ 3,
c) wenigstens einer aminofunktionellen Verbindung, die mindestens zwei (isocyanatreaktive) Aminogruppen aufweist und ausgewählt ist aus Ethylendiamin, IPDA und/oder H12-MDA
d) gegebenenfalls wenigstens ein Alkohol, der mindestens zwei Hydroxylgruppen und eine Molmasse ≥ 60 und ≤ 399 g/mol aufweist,
e) gegebenenfalls wenigstens eine Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Weiterhin bevorzugt ist der Polyurethanharnstoff in dieser vorgenannten Ausführungsform aufgebaut aus ≥ 5 und ≤ 60 Gew.-% der Komponente a), ≥ 30 und ≤ 90 Gew.-% der Komponente b), ≥ 2 und ≤ 25 Gew.-% der Komponente c), ≥ 0 und ≤ 10 Gew.-% der Komponente d), ≥ 0 und ≤ 10 Gew.-% der Komponente e) und ≥ 0 und ≤ 20 Gew.-% der Komponente f), jeweils bezogen auf die Gesamtmasse des Polyurethanharnstoffs, wobei sich die Komponenten a) bis f) zu 100 Gew.-% ergänzen.

Besonders bevorzugt ist der Polyurethanharnstoff in dieser vorgenannten Ausführungsform aufgebaut aus ≥ 10 und ≤ 40 Gew.-% der Komponente a), ≥ 55 und ≤ 85 Gew.-% der Komponente b), ≥ 5 und ≤ 20 Gew.-% der Komponente c), ≥ 0 und ≤ 3 Gew.-% der Komponente d), ≥ 0 und ≤ 3 Gew.-% der Komponente e) und ≥ 0 und ≤ 1 Gew.-% der Komponente f), jeweils bezogen auf die Gesamtmasse des Polyurethanharnstoffs, wobei sich die Komponenten a) bis f) zu 100 Gew.-% ergänzen.

In einer besonders bevorzugten Ausführungsform der Erfindung ist der erfindungsgemäß verwendete Polyurethanharnstoff aufgebaut aus
a) wenigstens einem Diisocyanat ausgewählt aus IPDI und/oder H12-MDI,
b) wenigstens einem Polyetherpolyol mit einem zahlenmittleren Molekulargewicht Mn ≥ 500 und ≤ 2500 g/mol und einer Hydroxylfunktionalität von ≥ 1,9 und ≤ 3, ausgewählt aus Polypropylenglykolen und/oder Poly(tetramethylenglykol)polyetherpolyolen,
c) wenigstens einer aminofunktionellen Verbindung, ausgewählt aus IPDA und/oder H12-MDA,
d) gegebenenfalls wenigstens ein Alkohol, der mindestens zwei Hydroxylgruppen und eine Molmasse ≥ 60 und ≤ 399 g/mol aufweist,
e) gegebenenfalls wenigstens eine Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

Weiterhin bevorzugt ist der Polyurethanharnstoff in dieser vorgenannten Ausführungsform aufgebaut aus ≥ 5 und ≤ 60 Gew.-% der Komponente a), ≥ 30 und ≤ 90 Gew.-% der Komponente b), ≥ 2 und ≤ 25 Gew.-% der Komponente c), ≥ 0 und ≤ 10 Gew.-% der Komponente d), ≥ 0 und ≤ 10 Gew.-% der Komponente e) und ≥ 0 und ≤ 20 Gew.-% der Komponente f), jeweils bezogen auf die Gesamtmasse des Polyurethanharnstoffs, wobei sich die Komponenten a) bis f) zu 100 Gew.-% ergänzen.

Besonders bevorzugt ist der Polyurethanharnstoff in dieser vorgenannten Ausführungsform aufgebaut aus ≥ 10 und ≤ 40 Gew.-% der Komponente a), ≥ 55 und ≤ 85 Gew.-% der Komponente b), ≥ 5 und ≤ 20 Gew.-% der Komponente c), ≥ 0 und ≤ 3 Gew.-% der Komponente d), ≥ 0 und ≤ 3 Gew.-% der Komponente e) und ≥ 0 und ≤ 1 Gew.-% der Komponente f), jeweils bezogen auf die Gesamtmasse des Polyurethanharnstoffs, wobei sich die Komponenten a) bis f) zu 100 Gew.-% ergänzen.

Bevorzugt ist der Polyurethanharnstoff ausschließlich aufgebaut aus den Komponenten a) bis c) und gegebenenfalls d) bis f), besonders bevorzugt ausschließlich aus den Komponenten a) bis c).

Vorteilhaft weist der Polyurethanharnstoff ein zahlenmittleres Molekulargewicht Mn ≥ 2000 und ≤ 50000 g/mol, besonders vorteilhaft ≥ 3000 und ≤ 20000 g/mol, auf.

Der Polyurethanharnstoff wird bevorzugt hergestellt, indem die Komponenten a) und b) sowie gegebenenfalls d) und f) in einem ersten Schritt zu einem NCO-terminierten Präpolymer umgesetzt werden, welches dann in einem darauf folgenden Schritt mit der Komponente c) sowie gegebenenfalls den Komponenten d) und e) umgesetzt wird.

Für die Herstellung der Polyurethanharnstoffe werden bevorzugt die Komponenten a) und b) sowie gegebenenfalls d) und f) zur Herstellung eines NCO-terminierten Präpolymers ganz oder teilweise vorgelegt, gegebenenfalls mit einem gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden. In einer bevorzugten Variante wird jedoch ohne den Zusatz von Urethanisierungs-Katalysatoren gearbeitet.

Anschließend werden die gegebenenfalls zu Beginn der Reaktion noch nicht zugegebenen Bestandteile von a) und b) sowie gegebenenfalls d) und f) zudosiert.

Bei der Herstellung der NCO-terminierten Präpolymere aus den Komponenten a) und b) sowie gegebenenfalls d) und f) beträgt das Stoffmengenverhältnis von Isocyanat-Gruppen zu isocyanatreaktiven Gruppen im allgemeinen ≥ 1,05 und ≤ 3,5, bevorzugt ≥ 1,1 und ≤ 3,0, besonders bevorzugt ≥ 1,1 und ≤ 2,5.

Unter isocyanatreaktiven Gruppen sind alle gegenüber Isocyanat-Gruppen reaktiven Gruppen zu verstehen, wie beispielsweise primäre und sekundäre Aminogruppen, Hydroxygruppen oder Thiolgruppen.

Die Umsetzung der Komponenten a) und b) sowie gegebenenfalls d) und f) zum Präpolymer erfolgt teilweise oder vollständig, bevorzugt aber vollständig. Es werden so Polyurethan-Präpolymere, die freie Isocyanatgruppen enthalten, in Substanz oder in Lösung erhalten.

Bevorzugt wir das NCO-terminierte Präpolymer ausschließlich aus den Komponenten a) und b) hergestellt.

Im Anschluss wird bevorzugt in einem weiteren Verfahrensschritt, falls noch nicht oder nur teilweise geschehen, das erhaltene Präpolymer mit Hilfe von einem oder mehreren organischen Lösemitteln gelöst. Als Lösemittel wird dabei bevorzugt ebenfalls ein Lösemittel oder Lösemittelgemisch eingesetzt, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird. Für das Lösemittel und das Lösemittelgemisch gelten ebenfalls die nachstehenden bevorzugten Ausführungsformen zum Lösemittel oder Lösemittelgemisch in dem der Polyurethanharnstoff gelöst ist. Das Lösemittel oder Lösemittelgemisch kann dabei auch von dem Lösemittel oder Lösemittelgemisch, in dem der Polyurethanharnstoff als Endprodukt später gelöst ist, verschieden sein. Bevorzugt ist das Lösemittel oder Lösemittelgemisch identisch mit dem Lösemittel oder Lösemittelgemisch, in dem der Polyurethanharnstoff als Endprodukt später gelöst ist.

Bevorzugt besteht das bei der Herstellung eingesetzte Lösemittel aus einem oder mehreren monohydroxyfunktionellen Alkoholen.

Das Verhältnis von Lösemittel zu Präpolymer liegt dabei bevorzugt bei ≥ 1:10 und ≤ 5:1, besonders bevorzugt bei ≥ 1:2 und ≤ 2:1 Gewichtsteilen.

Das Präpolymer wird bevorzugt vor dem Lösen abgekühlt auf Temperaturen von -20 bis 60°C, vorzugsweise 0 bis 50°C und besonders bevorzugt von 15 bis 40°C.

In einem, sich gegebenenfalls an das Lösen des NCO-terminierten Präpolymers anschließenden, weiteren Schritt wird bevorzugt dann das im ersten Schritt erhaltene NCO-terminierte Präpolymer ganz oder teilweise mit der Komponente c) sowie gegebenenfalls den Komponenten d) und e) umgesetzt. Diese Umsetzung wird im allgemeinen als Kettenverlängerung, bzw. im Fall der Komponente e) als Kettenabbruch bezeichnet.

Bevorzugt wird dabei das NCO-terminierte Präpolymer vorgelegt und die Komponenten c) sowie gegebenenfalls d) und e) zudosiert. Bevorzugt erfolgt zunächst eine teilweise Umsetzung der NCO-Gruppen des Präpolymers mit den Komponenten c) und gegebenenfalls d) und im Anschluss der Kettenabbruch durch Umsetzung der verbleibenden NCO Gruppen mit der Komponente e). Die Komponenten c) und gegebenenfalls e) können dabei auch stufenweise in mehreren Schritten, insbesondere in zwei Schritten, zugegeben werden.

Die Komponente c) sowie gegebenenfalls d) und e) werden bevorzugt gelöst in einem oder mehreren organischen Lösemitteln eingesetzt. Als Lösemittel wird dabei bevorzugt ebenfalls ein Lösemittel oder Lösemittelgemisch eingesetzt, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird. Für das Lösemittel und das Lösemittelgemisch gelten ebenfalls die nachstehenden bevorzugten Ausführungsformen zum Lösemittel oder Lösemittelgemisch in dem der Polyurethanharnstoff gelöst ist.

Das Lösemittel oder Lösemittelgemisch kann dabei auch von dem Lösemittel oder Lösemittelgemisch, in dem der Polyurethanharnstoff als Endprodukt später gelöst ist, verschieden sein. Bevorzugt ist das Lösemittel oder Lösemittelgemisch identisch mit dem Lösemittel oder Lösemittelgemisch, in dem der Polyurethanharnstoff als Endprodukt später gelöst ist.

Bevorzugt besteht das bei der Herstellung eingesetzte Lösemittel für Komponente c) aus einem oder mehreren monohydroxyfunktionellen Alkoholen.

Wenn Lösemittel als Verdünnungsmittel verwendet werden, so beträgt der Verdünnungsmittelgehalt in der bei der Kettenverlängerung eingesetzten Komponenten c), sowie gegebenenfalls d) und e) bevorzugt 1 bis 95 Gew.-%, besonders bevorzugt 3 bis 50 Gew.-%, bezogen auf das Gesamtgewicht der Komponente c) sowie gegebenenfalls d) und e) einschließlich Verdünnungsmittel.

Die Zugabe der Komponenten c) sowie gegebenenfalls d) und e) erfolgt bevorzugt bei Temperaturen von -20 bis 60°C, vorzugsweise 0 bis 50 und besonders bevorzugt von 15 bis 40°C.

Der Kettenverlängerungsgrad, also das molare Verhältnis von NCO-reaktiven Gruppen der zur Kettenverlängerung und Kettenabbruch eingesetzten Komponenten c) sowie gegebenenfalls d) und e) zu freien NCO-Gruppen des Präpolymers, liegt im allgemeinen ≥ 50 und ≤ 120%, besonders bevorzugt ≥ 60 und ≤ 100% und ganz besonders bevorzugt ≥ 70 und ≤ 95%.

Bevorzugt beträgt das molare Verhältnis der isocyanatreaktiven Gruppen der Komponente c) zu den freien NCO-Gruppen des Präpolymers ≥ 50 und ≤ 120%, besonders bevorzugt ≥ 60 und ≤ 100% und ganz besonders bevorzugt ≥ 70 und ≤ 95%.

In einer bevorzugten Ausführungsform der Erfindung werden die freien NCO-gruppen des Präpolymers nur teilweise mit der Komponente c) umgesetzt, bevorzugt beträgt dabei das molare Verhältnis der isocyanatreaktiven Gruppen der Komponente c) zu den freien NCO-Gruppen des Präpolymers ≥ 60 und ≤ 95% und die verbleibenden freien NCO Gruppen reagieren mit den Hydroxygruppen des Lösemittels ab, so dass ein NCO freier Polyurethanharnstoff entsteht.

Im Anschluss an die Herstellung kann der Polyurethanharnstoff, sofern im Herstellungsverfahren bereits erfindungsgemäße Lösemittel oder Lösemittelgemische eingesetzt wurden, weiterhin mit einem Lösemittel oder Lösemittelgemisch verdünnt und dabei gelöst werden, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunk-tionellen Alkohol enthält, verwendet wird.

Wurden während der Umsetzung keine erfindungsgemäßen Lösemittel oder Lösemittelgemische eingesetzt, so wird im Anschluss an die Herstellung des Polyurethanharnstoffs dieser in einem Lösemittel oder Lösemittelgemisch eingesetzt, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird.

Das Lösen des Polyurethanharnstoffs kann mit den üblichen Techniken zur Scherung erfolgen, beispielsweise durch Rühren mit Standard-Rührern, beispielsweise wie in DIN 28131 genannt.

Das Lösen des Polyurethanharnstoffs erfolgt vorzugsweise ohne die zusätzliche Zugabe von externen Emulgatoren. Die erfindungsgemäße verwendeten Polyurethanharnstofflösungen umfassen vorzugsweise keine externen Emulgatoren.

Als Lösemittel oder Bestandteil des Lösemittelgemisches geeignet sind prinzipiell alle monohydroxyfunktionellen, aliphatischen Alkohole mit einem bis sechs Kohlenstoffatomen, wie beispielsweise Methanol, Ethanol, n-Propanol, iso-Propanol, n-Butanol, sec.-Butanol und/oder Butylglykol. Besonders bevorzugt handelt es sich bei dem monohydroxyfunktionellen Alkohol um Ethanol. Wird ein Lösemittelgemisch eingesetzt, so können neben den monohydroxyfunktionellen Alkoholen noch ≤ 50 Gew.-%, bezogen auf die Gesamtmasse des Lösemittelgemisches, eines weiteren organischen Lösemittels eingesetzt werden. Geeignete Lösemittel sind dabei sind beispielsweise Ester, wie z.B. Ethylacetat, Butylacetat, Methyoxypropylacetat oder Butyrolacton, Ketone, wie z.B. Aceton oder Methylethylketon, Ether, wie z.B. Tetrahydrofuran oder tert.-Butylmethylether, aromatische Lösungsmittel, wie z.B. Xylol oder Solvent Naphtha. Im Falle der Verwendung von Ethanol können typische Vergällungsmittel als Additive in den üblichen Zuschlagmengen enthalten sein.

Bevorzugt beträgt der Anteil der weiteren organischen Lösemittel ≤ 30 Gew.-%, besonders bevorzugt ≤ 5 Gew.-% und ganz besonders bevorzugt ≤ 2 Gew.-%. In einer ganz besonders bevorzugten Ausführungsform sind neben monohydroxyfunktionellen, aliphatischen Alkoholen keinerlei weitere organische Lösemittel enthalten.

Als weitere Lösemittel ungeeignet sind physiologisch unverträgliche Lösemittel wie beispielsweise Dimethylformamid, N-Methylpyrrolidon oder Toluol, wie sie oft als Colösemittel für Polyurethane oder Polyurethanharnstoffe eingesetzt werden, das diese vorzugsweise nicht in kosmetischen Zusammensetzungen enthalten sein sollten.

Bei den weiteren Lösemitteln handelt es sich auch nicht um Wasser. Die durch Lösen des Polyurethanharnstoffs in den erfindungsgemäß verwendeten Lösemitteln oder Lösemittelgemischen erhaltene Polyurethanharnstofflösung ist bevorzugt wasserfrei, ausgenommen der Anteile an Wasser, die in den eingesetzten organischen Lösemitteln herstellungsbedingt enthalten sind.

Der Wasseranteil der Polyurethanharnstofflösung liegt bevorzugt bei ≤ 10 Gew.-%, besonders bevorzugt bei ≤ 4,5 Gew.-% und ganz besonders bevorzugt bei ≤ 1 Gew.-%, bezogen auf die Gesamtmasse der Polyurethanharnstofflösung.

Der Anteil des Polyurethanharnstoffs (als Aktivstoff) an der erfindungsgemäß eingesetzten Polyurethanharnstofflösung (auch bezeichnet als Feststoffgehalt) liegt bevorzugt ≥ 10 und ≤ 80 Gew.-%, besonders bevorzugt ≥ 15 und ≤ 60 Gew.-% und ganz besonders bevorzugt bei ≥ 20 und ≤ 50 Gew.-%.

Ein weiterer Gegenstand der Erfindung ist die erfindungsgemäße Sonnenschutz-Zusammensetzung zum Schutz der Haut und Haare vor negativen Wirkungen der Sonnenstrahlung.

Die erfindungsgemäßen Sonnenschutz-Zusammensetzungen liegen bevorzugt in Form von Gelen, Ölen, Sprays und Aerosolen vor, die vorzugsweise transparent sind. Transparent im Sinne dieser Erfindung bedeutet, dass die Trübungswerte der Zusammensetzung ≤ 100 NTU (Nephelometric Turbidity Unit), bevorzugt ≤ 50 NTU, besonders bevorzugt ≤ 10 NTU und ganz besonders bevorzugt ≤ 5 NTU sind. Die Trübungswerte werden dabei durch eine Streulichtmessung im 90°-Winkel (Nephelometrie) bei einer Wellenlänge der Mess-Strahlung 860 nm entsprechend DIN EN ISO 7027 bestimmt, durchgeführt bei 23°C mit einem Labortrübungsmessgerät Modell 2100AN der Firma HACH LANGE GmbH, Berlin, Deutschland.

Die Sonnenschutz-Zusammensetzungen können ferner mit einem Treibgas aufgeschäumt werden.

Der Anteil der in der Sonnenschutz-Zusammensetzung eingesetzten Polyurethanharnstofflösung beträgt bevorzugt ≥ 0,5 und ≤ 80 Gew.-%, besonders bevorzugt ≥ 1 und ≤ 60 Gew.-% und ganz besonders bevorzugt ≥ 2 und ≤ 40 Gew.-%, bezogen auf die Gesamtmasse der Sonnenschutz-Zusammensetzung.

Der Feststoffgehalt der Polyurethanharnstofflösung ist dabei bevorzugt so gewählt, dass die kosmetischen Zusammensetzungen bevorzugt ≥ 0,1 Gew.-% und ≤ 30 Gew.-%, besonders bevorzugt ≥ 0,5 und ≤ 20 Gew.-% und ganz besonders bevorzugt ≥ 1 und ≤ 10 Gew.-% des Polyurethanharnstoffs als Aktivstoff, bezogen auf die Gesamtmasse der Sonnenschutz-Zusammensetzung, enthalten.

Als Aktivstoff wird der Polyurethanharnstoff ohne Lösemittel oder Lösemittelgemisch verstanden.

Die erfindungsgemäßen Sonnenschutz-Zusammensetzungen weisen bevorzugte eine Viskosität von ≥ 2 und ≤ 20000 mPas auf. Gel- oder Lotionsförmige Zusammensetzungen weisen besonders bevorzugt eine Viskosität von ≥ 1000 und ≤ 20000 mPas und ganz besonders bevorzugt von ≥ 2000 und ≤ 10000 mPas auf. Sprühbare Zusammensetzungen wie Sonnensprays weisen besonders bevorzugt eine Viskosität von ≥ 2 und ≤ 2000 mPas und ganz besonders bevorzugt von ≥ 5 und ≤ 500 mPas auf.

Die angegebenen Viskositäten werden mittels Rotationsviskosimetrie nach DIN 53019 bei 23°C mit einem Rotationsviskosimeter der Firma Anton Paar Germany GmbH, Ostfildern, DE, bei einer Scherrate von 10 s⁻¹ bestimmt.

Die erfindungsgemäßen können in unterschiedlicher Konsistenz vorliegen, halbfest, insbesondere als Gele oder dünnflüssig, insbesondere als sprühbare Zusammensetzungen, Aerosole oder Öle.

Bevorzugt handelt es sich bei den Sonnenschutz- Zusammensetzungen um solche die überwiegend Alkohol basiert sind, also ≥ 10 und ≤ 90 Gew.-%, bezogen auf die Gesamtmasse der kosmetischen Zusammensetzung, bevorzugt ≥ 15 und ≤ 70 Gew.-% und besonders bevorzugt ≥ 20 und ≤ 60 Gew.-% an aliphatischen Alkoholen, mit 1 bis 6 Kohlenstoffatomen, bevorzugt 1 bis 4 Kohlenstoffatomen, enthalten. Die Alkohole sind dabei bevorzugt ausgewählt aus Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, oder deren Gemischen. Besonders bevorzugt enthalten die Alkohole Ethanol oder bestehen daraus, ganz besonders bevorzugt handelt es sich bei dem verwendeten Alkohol um Ethanol.

Besonders bevorzugt handelt es sich bei den Sonnenschutz-Zusammensetzungen um alkoholische Lösungen.

Die Sonnenschutz-Zusammensetzungen enthalten bevorzugt einen Wasseranteil von ≥ 0 und ≤ 30 Gew.-%, besonders bevorzugt von ≥ 0 und ≤ 20 Gew.-%, ganz besonders bevorzugt von ≥ 0 und ≤ 5 Gew.-% und weiterhin bevorzugt ≥ 0 und < 2 Gew.-%. Insbesondere bevorzugt sind die Sonnenschutz-Zusammensetzungen wasserfrei, enthalten somit nicht mehr Wasser, als das was herstellungsbedingt über die Rohstoffe unvermeidbar in die Formulierung eingebracht wird.

Das verwendete Wasser in der erfindungsgemäßen Zusammensetzung kann ein Blütenwasser, reines demineralisiertes Wasser, Mineralwasser, Thermalwasser und/oder Meerwasser sein.

Die erfindungsgemäße Sonnenschutz-Zusammensetzung enthält bevorzugt insgesamt ≥ 1 und ≤ 40 Gew.-%, besonders bevorzugt ≥ 5 und ≤ 35 Gew.-% und ganz besonders bevorzugt ≥ 10 und ≤ 30 Gew.-% Sonnenschutzfilter-Substanzen, bezogen auf das Gesamtgewicht der Sonnenschutz-Zusammensetzung. Bei der angegebenen Menge handelt es sich um die Summe der Mengen aller in der erfindungsgemäßen Sonnenschutz-Zusammensetzung enthaltenen Sonnenschutzfilter-Substanzen. Sonnenschutzfilter-Substanzen können auch als Sonnenschutzfilter oder Sonnenschutz vermittelnde Substanzen bezeichnet werden.

Bei den Sonnenschutzfiltern handelt es sich insbesondere um UV-Filter, welche Licht im UV-Wellenlängenbereich insbesondere von weniger als 400 nm filtern. Die Begriffe Sonnenschutzfilter-Substanzen und UV-Filter-Substanzen werden im Rahmen dieser Anmeldung als äquivalente Begriffe verwendet.

Üblicherweise unterteilt man den UV-Wellenlängenbereich wie folgt:

| UV-Licht | Wellenlängenbereich in nm |
|---|---|
| Nahes UV | 400-200 nm |
| UV-A | 380-315 nm |
| UV-B | 315-280 nm |
| UV-C | 280-100 nm |
| Fernes UV, Vakuumstrahlung | 200-10 nm |
| Extremes UV | 31-1 nm |

Die Sonnenschutzfilter (oder UV-Filter) können unter den organischen Filtern, den physikalischen Filtern und/oder deren Gemischen ausgewählt werden.

Die erfindungsgemäße Sonnenschutz-Zusammensetzung kann insbesondere UV-A-Filter, UV-B-Filter, Breitbandfilter und/oder physikalische Filter als Sonnenschutzfilter-Substanzen enthalten. Die erfindungsgemäße Sonnenschutz-Zusammensetzung enthält vorzugsweise Mischungen von wenigstens zwei dieser vorgenannten Arten von Sonnenschutzfilter-Substanzen. Die erfindungsgemäße Sonnenschutz-Zusammensetzung kann auch mehrere Sonnenschutzfilter-Substanzen enthalten, die einer dieser Arten von Sonnenschutzfilter-Substanzen zugeordnet werden, d.h. z.B. mehrere UV-A-Filter und/oder mehrere UV-B-Filter. Hierbei sind beliebige Kombinationen möglich.

Die eingesetzten UV-Filter können öllöslich oder wasserlöslich sein. Die folgende Liste der genannten UV-Filter ist selbstverständlich nicht limitierend.

Unter den UV-B-Filtern sind beispielsweise zu nennen:
- (1) Salicylsäurederivate, besonders Homomenthylsalicylat, Octylsalicylat und Salicylsäure(4-isopropylbenzyl)ester;
- (2) Zimtsäurederivate, insbesondere 2-Ethylhexyl-p-methoxycinnamat, das von der Firma Givaudan unter der Bezeichnung Parsol MCX^{®} erhältlich ist und 4-Methoxyzimtsäureisopentylester;
- (3) flüssige β,β'-Diphenylacrylatderivate, insbesondere 2-Ethylhexyl α,β'-diphenylacrylat oder Octocrylen, das von der Firma BASF unter der Bezeichnung UVINUL N539^{®} erhältlich ist;
- (4) p-Aminobenzoesäurederivate, insbesondere 4-(dimethylamino)-benzoesäure (2-ethylhexyl)ester, 4-(dimethylamino)benzoesäureamylester;
- (5) 3-Benzylidencampher-Derivate, insbesondere 3-(4-Methylbenzyliden)campher der von der Firma Merck unter der Bezeichnung EUSOLEX 6300^{®} im Handel ist, 3-Benzylidencampher, Benzylidencampher sulfonsäure und Polyacrylamidomethyl Benzylidencampher;
- (6) 2-Phenylbenzimidazol-5-sulfonsäure, die unter der Bezeichnung EUSOLEX 232^{®} von Merck erhältlich ist; oder durch organische Amine neutralisierte 2-Phenylbenzimidazol-5-sulfonsäure, wie in der DE 20 2010 006 005 U1 beschrieben;(7) 1,3,5-Triazinderivate, insbesondere: -2,4,6-Tris[p-(2'-ethylhexyl-1'-oxycarbonyl)anilino]-1,3,5-triazin, das von der Firma BASF unter der Bezeichnung UVINUL T150^{®} angeboten wird, und - Dioctylbutamidotriazon, das von der Firma Sigma 3V unter der Bezeichnung UVASORB HEB^{®} angeboten wird;
- (8) Ester der Benzalmalonsäure, insbesondere 4-Methoxybenzalmalonsäuredi(2-ethylhexyl)ester und 3-(4-(2,2-bis ethoxycarbonylvinyl)-phenoxy)propenyl)-methoxysiloxan/ dimethylsiloxan-Copolymer, das von der Firma Roche Vitamines unter der Bezeichnung Parsol^{®} SLX erhältlich ist; und
- (9) die Gemische dieser Filter.

Besonders bevorzugt zeigen die Sonnenschutz-Zusammensetzungen einen SPF Boosting-Effekt, d.h. die erfindungsgemäße Sonnenschutz-Zusammensetzung enthaltend die erfindungsgemäße Polyurethanlösung und Sonnenschutzfilter-Substanzen weist einen deutlich höherem SPF auf als die Mischung der Sonnenschutzfilter-Substanzen alleine.

Die erfindungsgemäße Sonnenschutz-Zusammensetzung enthält bevorzugt wenigstens einen UV-B-Filter, besonders bevorzugt Octocrylen, in einer Menge ≥ 4 und ≤ 12 Gew.-%, bevorzugt ≥ 5 und ≤ 12 Gew.-%, besonders bevorzugt ≥ 6 und ≤ 12 Gew.-%, ganz besonders bevorzugt ≥ 7 und ≤ 11 Gew.-%, bezogen auf das Gesamtgewicht der Sonnenschutz-Zusammensetzung.

Als UV-A-Filter sind beispielsweise zu nennen:
- (1) Dibenzoylmethanderivate, besonders das 4-(t-Butyl)-4'-methoxydibenzoylmethan, das von der Firma Givaudan unter der Bezeichnung PARSOL 1789^{®} angeboten wird und 1 -Phenyl-3-(4' -isopropylphenyl)propan-1 ,3-dion;
- (2) Benzol-1,4-[di(3-methylidencampher-10-sulfonsäure)], gegebenenfalls ganz oder teilweise neutralisiert, unter der Bezeichnung MEXORYL SX^{®} von Chimex im Handel.
- (3) 2-(4'-Diethylamino-2'-hydroxybenzoyl)-benzoesäurehexylester (auch Aminobenzophenon);
- (4) Silanderivate oder Polyorganosiloxane mit Benzophenongruppen;
- (5) Anthranilate, besonders Menthylanthranilat, das von der Firma Symrise unter der Bezeichnung NEO HELIOPAN MA^{®} angeboten wird;
- (6) Verbindungen, die pro Molekül mindestens zwei Benzoazolylgruppen oder mindestens eine Benzodiazolylgruppe enthalten, insbesondere 1,4-Bis-benzimidazolylphenylen-3,3',5,5'-tetrasulfonsäure sowie ihre Salze, die von der Firma Symrise im Handel sind;
- (7) Siliciumderivate von Benzimidazolylbenzazolen, die N-substituiert sind, oder von Benzofuranylbenzazolen, insbesondere:
   - 2-[1-[3-[1,3,3,3-Tetramethyl-1- [(trimethylsilyl)oxy] disiloxanyl] -propyl] -1H-benzimidazol-2-yl] -benzoxazol;
   - 2-[1-[3-[1,3,3,3-Tetramethyl-1-[(trimethylsilyl)oxy]disiloxanyl]-propyl]-1H-benzimidazol-2-yl] -benzothiazol;
   - 2-[1-(3-Trimethylsilanylpropyl)-1H-benzimidazol-2-yl]-benzoxazol;
   - 6-Methoxy-1,1'-bis(3-trimethylsilanylpropyl)1H,1'H-[2,2']dibenzimidazolylbenzoxazol;
   - 2-[1-(3-Trimethylsilanylpropyl)-1H-benzimidazol-2-yl]-benzothiazol;
      die in der Patentanmeldung EP-A-1 028 120 beschrieben sind;
- (8) Triazinderivate, insbesondere 2,4-bis-[5-1(dimethylpropyl)benzoxazol-2-yl-(4-phenyl)-imino]-6-(2-ethylhexy1)-imino-1,3,5-triazin, das von der Firma 3V unter der Bezeichnung Uvasorb^{®}K2A angeboten wird; und
- (9) deren Gemische.

In einer bevorzugten Ausführungsform enthält die erfindungsgemäße Sonnenschutz-Zusammensetzung als Sonnenschutzfilter-Substanz, vorzugsweise wenigstens einen UV-A-Filter, welcher bevorzugt ein Dibenzoylmethanderivat, besonders bevorzugt 4-(t-Butyl)-4'-methoxydibenzoylmethan ist. Dieses Dibenzoylmethanderivat, bevorzugt 4-(t-Butyl)-4'-methoxydibenzoylmethan ist vorzugsweise in einer Menge von ≥ 1 und ≤ 5 Gew.-%, bezogen auf das Gesamtgewicht der Sonnenschutz-Zusammensetzung, in der erfindungsgemäßen Sonnenschutz-Zusammensetzung enthalten.

Als geeignete Breitbandfilter sind beispielweise zu nennen:
- (1) Benzophenonderivate, beispielsweise
   - 2,4-Dihydroxybenzophenon (Benzophenon-1);
   - 2,2',4,4'-Tetrahydroxybenzophenon (Benzophenon-2);
   - 2-Hydroxy-4-methoxybenzophenon (Benzophenon-3), von der Firma BASF unter der Bezeichnung UNIVNUL M40^{®} erhältlich;
   - 2-Hydroxy-4-methoxybenzophenon-5-sulfonsäure (Benzophenon-4), sowie ihre Sulfonatform (Benzonphenon-5), von der Firma BASF unter der Bezeichnung UVINUL MS40^{®} im Handel;
   - 2,2'-Dihydroxy-4,4'-dimethoxybenzophenon (Benzophenon-6-);
   - 5-Chlor-2-hydroxybenzophenon (Benzophenon-7-);
   - 2,2'-Dihydroxy-4-methoxybenzophenon (Benzophenon-8);
   - das Dinatriumsalz von 2,2'-Dihydroxy-4,4'-dimethoxybenzophenon-5,5'-disulfonsäure (Benzophenon-9-);
   - 2-Hydroxy-4-methoxy-4'-methylbenzophenon (Benzophenon-10);
   - Benzophenon-11;
   - 2-Hydroxy-4-(octyloxy)-benzophenon (Benzophenon-12).
- (2) Triazinderivate, insbesondere das 2,4-Bis{[4-2-ethylhexyloxy)-2-hydroxy]-phenyl}-6-(4-methoxyphenyl)-1,3,5-triazin , das von der Firma Ciba Geigy unter der Bezeichnung TINOSORB S^{®} angeboten wird, und das 2,2'-Methylen-bis[6-(2H-benzotriazol-2-yl)4-(1,1,3,3-tetramethylbutyl)phenol], das von der Firma Ciba Geigy unter der Bezeichnung TINOSORB M^{®} erhältlich ist; und
- (3) 2-(1H-benzotriazol-2-yl)-4-methyl-6-[2-methyl-3-[1,3,3,3-tetramethyl-1 - [(trimethylsilyl)oxy]disiloxanyl]propyl]-phenol mit der INCI-Bezeichnung Drometrizole Trisiloxane.

Es können auch ein Gemisch von mehreren Filtern und ein Gemisch von UV-B-Filtern, UV-A-Filtern und Breitbandfilter sowie Gemische mit physikalischen Filtern verwendet werden.

Unter den physikalischen Filtern können beispielsweise die Sulfate des Bariums, Oxide von Titan (Titandioxid, amorph oder kristallin in Form von Rutil und/oder Anatas), von Zink, von Eisen, von Zirkonium, von Cer, von Silicium, von Mangan oder deren Gemische angegeben werden. Die Metalloxide können in Partikelform mit einer Größe im Mikrometerbereich oder Nanometerbereich (Nanopigmente) vorliegen. Die mittleren Partikelgrößen betragen für die Nanopigmente beispielsweise 5 bis 100 nm. In transparenten Zusammensetzungen werden bevorzugt physikalische Filter mit Partikelgrößen im Nanometerbereich oder keine physikalischen Filter eingesetzt.

Öllösliche UV-Filter können solche sein, die flüssig sind, insbesondere ölartig und selbst auch als Lösemittel für andere öllösliche UV-Filter dienen können oder solche die fest sind und in Ölen gelöst eingesetzt werden.

Bevorzugt eingesetzte, flüssige, öllösliche UV-Filter sind Octocrylen, Ethylhexylmethoxycinnamat, Ethylhexylsalicylat und/oder Homosalat.

Bevorzugt eingesetzte, feste, öllösliche UV-Filter sind Butylmethoxydibenzoylmethan (Avobenzone), Dioctylbutylamidotriazon (INCI: Diethylhexyl Butamidotriazone), 2 ,4-B is-{[ 4-(2-ethyl-hexyloxy)-2-hyd roxy]-phenyl}-6-(4-methoxyphenyl)-1,3,5-triazin (INCI: Bis-Ethylhexyloxyphenol Methoxyphenyl Triazin), Ethylhexyltriazon, DiethylaminoHydroxybenzoylHexylBenzoat. Darüber hinaus können auch alle anderen öllöslichen Filter die im Anhang VI der EU-Kosmetikverordnung (1223/2009) aufgeführt sind eingesetzt werden.

In einer bevorzugten Ausführungsform der Erfindung enthalten die kosmetischen Zusammensetzungen wenigstens einen flüssigen, öllöslichen UV-Filter.

In einer bevorzugten Ausführungsform der erfindungsgemäßen Sonnenschutzzusammensetzung enthält diese ≥ 5 und ≤ 35 Gew.-% Sonnenschutzfilter-Substanzen, bezogen auf das Gesamtgewicht der Sonnenschutz-Zusammensetzung, wovon wenigstens eine der Sonnenschutzfilter-Substanzen Benzophenon, ein Benzophenon-Derivat oder ein von Triazin abgeleitetes Derivat oder Octocrylen ist und diese Sonnenschutzfilter-Substanzen bevorzugt ≥ 4 und ≤ 12 Gew.-% des Gesamtgewicht der Sonnenschutz-Zusammensetzung ausmachen. Besonders bevorzugt ist wenigstens eine der Sonnenschutzfilter-Substanzen Octocrylen.

Die erfindungsgemäßen Sonnenschutz-Zusammensetzungen weisen in bevorzugten Ausführungsformen einen Sun-Protection-Factor (SPF) von mehr als 15, bevorzugt von mehr als 20 auf, gemessen nach der International Sun Protection Factor (SPF) test method nach COLIPA. Diese Messmethode ist dem Fachmann bekannt.

Die erfindungsgemäßen Sonnenschutz-Zusammensetzungen können weiterhin Öleund/oder Wachse enthalten, wobei es sich bei den Ölen um nicht flüchtige Öle und/oder flüchtige Öle handeln kann.

Die erfindungsgemäßen Sonnenschutz-Zusammensetzung enthält vorteilhaft ≥ 0,und ≤ 45 Gew.-% Öle, bezogen auf das Gesamtgewicht der Zusammensetzung, und besonders vorteilhaft ≥ 0,01 und ≤ 45 Gew.-% Öle und ganz besonders vorteilhaft ≥ 0,1 und ≤ 20 Gew:-% Öle.

Die nicht flüchtigen Öle werden vorteilhaft gewählt aus der Gruppe von mineralischer, tierischer, pflanzlicher oder synthetischer Herkunft, polaren und/oder unpolaren Ölen oder deren Gemischen.

Die polaren Öle werden vorteilhaft gewählt aus der Gruppe:
a) Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen,
b) Ester aus aromatischen Carbonsäuren und gesättigten und/oder ungesättigten, verzweigten und/oder unverzweigten Alkoholen einer Kettenlänge von 3 bis 30 C-Atomen.
   Solche Esteröle können dann vorteilhaft gewählt werden aus der Gruppe:
   Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, Isotridecylisononanoat, 2-Ethylhexylpalmitat, 2-Ethylhexyllaurat, 2-Ethylhexylisostearat, 2-Hexyldecylstearat, 2-Octyldodecylpalmitat, 2-Ethylhexylcocoat, Oleyloleat, Oleylerucat, Erucyloleat, Erucylerucat Dicaprypyp Carbonat (Cetiol^{®} CC) und Cocoglyceride (Myritol^{®} 331) sowie synthetische, halbsynthetische und natürliche Gemische solcher Ester, z.B. Jojobaöl.
c) Alkylbenzoate C12-15-Alkylbenzoat (Finsolv^{®} TN von Finetex) oder 2-Phenylethyl benzoate (X-Tend^{®} 226 von ISP)
d) Lecithine und den Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen ausgewählt werden. Beipielweise können die Fettsäuretriglyceride gewählt werden aus der Gruppe von Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Aprikosenkernöl, Avocadoöl und dergleichen mehr.
e) der Dialkylether und Dialkylcarbonate, vorteilhaft sind z.B. Dicaprylylether (Cetiol^{®} OE der Firma Cognis) und/oder Dicaprylylcarbonat (beispielsweise Cetiol^{®} CC der Firma Cognis).
f) gesättigten oder ungesättigten, verzweigten oder unverzweigten Alkohole, wie zum Beispiel Octyldodecanol.

Das nicht flüchtige Öl kann ebenfalls vorteilhaft auch ein unpolares Öl sein, welche gewählt wird aus der Gruppe der verzweigten und unverzweigten Kohlenwasserstoffe, insbesondere Mineralöl, Vaselineöl, Paraffinöl, Squalan und Squalen, Polyolefine beispielweise Polydecene, hydrogenierte Polyisobutene, C13-16 Isoparaffin und Isohexadecan.

Das unpolare nicht flüchtige Öl kann weiterhin unter den nicht flüchtigen Silikonölen ausgewählt werden.

Von den nicht flüchtigen Silikonölen können die Polydimethylsiloxane (PDMS), die gegebenenfalls phenyliert sind, wie die Phenyltrimethicon, oder gegebenenfalls substituiert sind mit aliphatischen und/oder aromatischen Gruppen oder mit funktionellen Gruppen, beispielsweise Hydroxygruppen, Thiolgruppen und/oder Aminogruppen; mit Fettsäuren, Fettalkoholen oder Polyoxyalkylenen modifizierte Polysiloxane und deren Gemische angegeben werden.

Besondere vorteilhafte Öle sind 2-Ethylhexylisostearat, Octyldodecanol, Isotridecylisononanoat, Isoeicosan, 2-Ethylhexylcocoat, C12-15 Alkylbenzoat, Caprylic/Capric Triglycerid, Dicaprylylether, Mineral Öl, Dicaprylyl Carbonate, Cocoglyceride, Butylene Glykol Dicarprylate/Dicaprate, Hydrogenated Polyisobutene, Cetaryl Isononanoate, Isodecyl Neopentanoate, Squalan, C13-16 Isoparaffin.

Die erfindungsgemäße Sonnenschutz-Zusammensetzung kann ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur von 30°C bis 200°C eine reversible Zustandsänderung fest/flüssig zeigt. Das Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Schellack, Mikrowachse, Ceresin, Ozokerit, Ouricuriwachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (Alkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die Wachse können in Mengen von ≥ 0 und ≤ 10 Gew.-%, bezogen auf das Gesamtgewicht der Zusammensetzung und vorzugsweise ≥ 0,01 und ≤ 10 Gew.-% und ganz besonders bevorzugt ≥ 0,1 und ≤ 5 Gew.-% enthalten sein.

Die erfindungsgemäße Sonnenschutz-Zusammensetzung kann ferner ein flüchtiges Öl enthalten, das aus der Gruppe von flüchtigen Kohlenwasserstoffölen, silikonierten Ölen oder fluorierten Ölen ausgewählt wird.

Die flüchtigen Öle können in einer Menge von ≥ 0 und ≤ 25 Gew.-%, bezogen auf das Gesamtgewicht der Zusammensetzung, vorzugsweise ≥ 0 und ≤ 20 Gew.-% und besonders bevorzugt ≥ 0,1 und ≤ 15 Gew.-% enthalten sein.

Im Sinne der vorliegenden Schrift ist ein flüchtiges Öl ein Öl, das im Kontakt mit der Haut bei Raumtemperatur und Atmosphärendruck in weniger als einer Stunde verdampft. Das flüchtige Öl ist bei Raumtemperatur flüssig und bei Raumtemperatur und Atmosphärendruck einen Dampfdruck von 0,13 bis 40 000 Pa (10⁻³ bis 300 mg Hg), vorzugsweise 1,3 bis 13 000 Pa (0,01 bis 100 mm Hg) und besonders bevorzugt 1,3 bis 13 00 Pa (0,01 bis 10 mm Hg) und einen Siedepunkt von 150 bis 260°C und vorzugsweise 170 bis 250°C besitzt.

Unter einem Kohlenwasserstofföl wird ein Öl verstanden, das im Wesentlichen aus Kohlenstoffatomen und Wasserstoffatomen und gegebenenfalls Sauerstoffatomen oder Stickstoffatomen gebildet wird und keine Siliziumatome oder Fluoratome enthält, wobei es auch aus Kohlenstoffatomen und Wasserstoffatomen bestehen kann; es kann Estergruppen, Ethergruppen, Aminogruppen oder Amidgruppen enthalten.

Unter einem silikonierten Öl wird ein Öl verstanden, das mindestens ein Siliziumatom und insbesondere Si-O-Gruppen enthält.

Unter einem fluorierten Öl ist ein Öl zu verstehen, das mindestens ein Fluoratom enthält.

Das erfindungsgemäß flüchtige Kohlenwasserstofföl kann unter den Kohlenwasserstoffölen mit einem Flammpunkt von 40 bis 102°C, vorzugsweise 40 bis 55°C und noch bevorzugter 40 bis 50°C ausgewählt werden.

Beispielweise sind die flüchtigen Kohlenwasserstofföle solche mit 8 bis 16 Kohlenstoffatomen und deren Gemische, insbesondere verzweigte C₈₋₁₆-Alkane, wie die Isoalkane (die auch als Isoparaffine bezeichnet werden) mit 8 bis 16 Kohlenstoffatomen, Isododecan, Isodecan, Isohexadecan und beispielsweise die Öle, die unter den Handelsnamen Isopars^{®} oder Permetyls^{®} angeboten werden; und die verzweigten C₈₋₁₆-Ester, wie Isohexylneopentanoat und deren Gemische.

Besonders vorteilhaft sind die flüchtigen Kohlenwasserstofföle wie Isododecan, Isodecan und Isohexadecan.

Das erfindungsgemäß flüchtige silikonierte Öl kann unter den silikonierten Ölen mit einem Flammpunkt von 40 bis 102°C, vorzugsweise einem Flammpunkt über 55°C und höchstens 95°C und besonders bevorzugt im Bereich von 65 bis 95°C ausgewählt werden.

Beispielweise sind die flüchtigen silikonierten Öle geradkettigen oder cyclischen Silikonöle mit 2 bis 7 Siliziumatomen genannt werden, wobei diese Silikone gegebenenfalls Alkyl- oder Alkoxygruppen mit 1 bis 10 Kohlenstoffatomen enthalten.

Besonders vorteilhaft sind die flüchtigen silikonierten Öle wie Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemische.

Das flüchtige fluorierte Öl besitzt im Allgemeinen keinen Flammpunkt.

Beispielweise sind die flüchtigen fluorierten Öle Nonafluorethoxybutan, Nonafluormethoxybutan, Decafluorpentan, Tetradecafluorhexan, Dodecafluorpentan und deren Gemische.

Die erfindungsgemäßen Sonnenschutz-Zusammensetzungen können zusätzlich ein oder mehrere weitere Zusatzstoffe enthalten, die in der Kosmetik üblich sind, wie Hilfs- und Zusatzmittel wie beispielsweise Emulgatoren, grenzflächenaktive Stoffe, Entschäumer, Verdicker, Tenside, Feuchthaltemittel, Füllstoff, Filmbildner, Lösemittel, Koaleszenzmittel, Gelbildner und/oder andere Polymerdispersionen wie beispielsweise Dispersionen auf Basis von Polyacrylaten, Füllstoffe, Weichmacher, Pigmente, Farbstoffe, Verlaufsmittel, Thixotropiemittel, Geschmeidigkeitsmittel, Konservierungsmittel, sensorische Additiv, Treibgas wie zum Beispiel Propan/Butan oder Dimethyl Ether etc.. Die Mengen der verschiedenen Zusatzstoffe sind dem Fachmann für den einzusetzenden Bereich bekannt und liegen vorzugsweise im Bereich von ≥ 0 und ≤ 40 Gew. -%, bevorzugt ≥ 0,01 und ≤ 40 Gew.- %, bezogen auf das Gesamtgewicht der Sonnenschutz-Zusammensetzung.

Die erfindungsgemäße Sonnenschutz-Zusammensetzung kann ein oder mehrere Feuchthaltemittel (Moisturizer) umfassen. Besonders vorteilhafte Feuchthaltemittel im Sinne der vorliegenden Erfindung sind beispielsweise Glycerin, Polyglycerin, Sorbit, Dimethylisosorbid, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches unter der Bezeichnung Fucogel^{™} 1000 von der Gesellschaft SOLABIA S.A. erhältlich ist.

In einer bevorzugten Ausführungsform der Erfindung enthält die Sonnenschutz-Zusammensetzung
I) wenigstens einen Polyurethanharnstoff, welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird,
II) wenigstens eine öllösliche Sonnenschutzfilter-Substanz,
III) wenigstens einen aliphatischen Alkohol mit 1 bis 6 C-Atomen.

In einer besonders bevorzugten Ausführungsform der Erfindung enthält die Sonnenschutz-Zusammensetzung
I) ≥ 0,5 und ≤ 80 Gew.-% wenigstens eines Polyurethanharnstoff, welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird,
II) ≥ 1 und ≤ 40 Gew.-% an Sonnenschutzfilter-Substanzen, enthaltend wenigstens eine öllöslichen Sonnenschutzfilter-Substanz,
III) ≥ 10 und ≤ 90 Gew.-% wenigstens eines aliphatischen Alkohols mit 1 bis 6 C-Atomen,
IV) ≥ 0 und ≤ 40 Gew.-% wenigstens eines Öl und/oder ≥ 0 und ≤ 10 Gew.-% wenigstens eines Wachses und
V) ≥ 0 und ≤ 40 Gew.-% für Sonnenschutz-Zusammensetzungen übliche weitere Additive, jeweils bezogen auf die Gesamtmasse der Sonnenschutz-Zusammensetzung, wobei die Komponenten I) bis V) sich zu 100 Gew.-% ergänzen.

Dabei ist die Komponente IV) bevorzugt nur dann = 0 wenn die Komponente B) bereits wenigstens eine flüssige, öllösliche Sonnenschutzfilter-Substanz enthält. Diese kann dann als Öl angesehen werden, das auch weitere feste öllösliche Sonnenschutzfilter-Substanzen lösen kann.

Der Feststoffgehalt der Polyurethanharnstofflösung ist dabei bevorzugt so gewählt, dass die Sonnenschutz-Zusammensetzung bevorzugt ≥ 0,5 und ≤ 20 Gew.-% des Polyurethanharnstoffs als Aktivstoff, bezogen auf die Gesamtmasse der kosmetischen Formulierung, enthalten.

In einer ganz besonders bevorzugten Ausführungsform der Erfindung enthält die Sonnenschutz - Zusammensetzung
I) ≥ 2 und ≤ 40 Gew.-% wenigstens eines Polyurethanharnstoff, welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird,
II) ≥ 10 und ≤ 30 Gew.-% an Sonnenschutzfilter-Substanzen, enthaltend wenigstens eine öllöslichen Sonnenschutzfilter-Substanz,
III) ≥ 15 und ≤ 70 Gew.-% wenigstens eines aliphatischen Alkohols mit 1 bis 4 C-Atomen,
IV) ≥ 0 und ≤ 20 Gew.-% wenigstens eines Öl und/oder 0 und ≤ 5 Gew.-% wenigstens eines Wachses und
V) ≥ 0 und ≤ 40 Gew.-% für Sonnenschutz-Zusammensetzungen übliche weitere Additive, jeweils bezogen auf die Gesamtmasse der Sonnenschutz -Zusammensetzung, wobei die Komponenten I) bis V) sich zu 100 Gew.-% ergänzen.

Dabei ist die Komponente IV) bevorzugt nur dann = 0 wenn die Komponente B) bereits wenigstens eine flüssige, öllösliche Sonnenschutzfilter-Substanz enthält. Diese kann dann als Öl angesehen werden, das auch weitere feste öllösliche Sonnenschutzfilter-Substanzen lösen kann.

Der Feststoffgehalt der Polyurethanharnstofflösung ist dabei bevorzugt so gewählt, dass die Sonnenschutz-Zusammensetzung bevorzugt ≥ 1 und ≤ 10 Gew.-% des Polyurethanharnstoffs als Aktivstoff, bezogen auf die Gesamtmasse der Sonnenschutz-Zusammensetzung, enthalten.

Gegenstand der Erfindung ist eine Sonnenschutz-Zusammensetzung enthaltend wenigstens eine Sonnenschutzfilter-Substanz und wenigstens einen Polyurethanharnstoff, welcher keine ionisch hydrophilierenden Gruppen aufweist und welcher gelöst in einem Lösemittel oder Lösemittelgemisch eingesetzt wird, wobei das Lösemittel ausschließlich aus einem oder mehreren monohydroxyfunktionellen Alkoholen besteht oder ein Lösemittelgemisch bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, verwendet wird.

Der verwendete Polyurethanharnstoff ist dabei aufgebaut aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b).

In einer ersten bevorzugten Ausführungsform der erfindungsgemäßen Sonnenschutz-Zusammensetzung weist der Polyurethanharnstoff keine hydrophilierenden Gruppen auf.

Eine zweite bevorzugte Ausführungsform der Erfindung umfasst eine Sonnenschutz-Zusammensetzung gemäße einer der vorstehend genannten Ausführungsformen der Erfindung, dadurch gekennzeichnet, dass die Komponente b) ausgewählt ist aus Poly(tetramethylenglykol)polyetherpolyolen.

Eine dritte bevorzugte Ausführungsform der Erfindung umfasst eine Sonnenschutz-Zusammensetzung gemäß einer der vorstehend genannten Ausführungsformen der Erfindung, dadurch gekennzeichnet, dass die Komponente b) eine zahlenmittlere Molekulargewicht Mn ≥ 500 und ≤ 2500 g/mol und eine Hydroxylfunktionalität von ≥ 1,9 und ≤ 3 aufweist.

Eine vierte bevorzugte Ausführungsform der Erfindung umfasst eine Sonnenschutz-Zusammensetzung gemäß einer der vorstehend genannten Ausführungsformen der Erfindung, dadurch gekennzeichnet, dass die Komponente a) ausgewählt ist aus aliphatischen, araliphatischen und/oder cycloaliphatischen Diisocyanaten, welche mindestens eine Isocyanatgruppe aufweisen, die an ein tertiäres C-Atom gebunden ist.

Eine fünfte bevorzugte Ausführungsform der Erfindung umfasst eine Sonnenschutz-Zusammensetzung gemäß einer der vorstehend genannten Ausführungsformen der Erfindung, dadurch gekennzeichnet, dass die Komponente a) ausgewählt ist aus IPDI und/oder H12-MDI.

Eine sechste bevorzugte Ausführungsform der Erfindung umfasst eine Sonnenschutz-Zusammensetzung gemäß einer der vorstehend genannten Ausführungsformen der Erfindung, dadurch gekennzeichnet, dass die Komponente c) ausgewählt ist aus Aminen, die mindestens zwei Aminogruppen aufweisen, die an primäre und/oder sekundäre C-Atome gebunden sind.

Eine siebte bevorzugte Ausführungsform der Erfindung umfasst eine Sonnenschutz-Zusammensetzung gemäß einer der vorstehend genannten Ausführungsformen der Erfindung, dadurch gekennzeichnet, dass die Komponente c) ausgewählt ist aus symmetrisch aufgebauten Diaminen.

Eine achte bevorzugte Ausführungsform der Erfindung umfasst eine Sonnenschutz-Zusammensetzung gemäß einer der vorstehend genannten Ausführungsformen der Erfindung, dadurch gekennzeichnet, dass die Komponente c) ausgewählt ist aus Ethylendiamin und/oder H12-MDA.

Eine neunte bevorzugte Ausführungsform der Erfindung umfasst eine Sonnenschutz-Zusammensetzung gemäß einer der vorstehend genannten Ausführungsformen der Erfindung, dadurch gekennzeichnet, dass die monohydroxyfunktionellen Alkohole ausgewählt sind aus aliphatischen Alkohole mit einem bis sechs Kohlenstoffatomen.

Eine zehnte bevorzugte Ausführungsform der Erfindung umfasst eine Sonnenschutz-Zusammensetzung gemäß einer der vorstehend genannten Ausführungsformen der Erfindung, dadurch gekennzeichnet, dass die Sonnenschutzfilter-Substanzen zu ≥ 5 und ≤ 35 Gew.-%, bezogen auf das Gesamtgewicht der Sonnenschutz-Zusammensetzung, enthalten sind.

Eine elfte bevorzugte Ausführungsform der Erfindung umfasst eine Sonnenschutz-Zusammensetzung gemäß einer der vorstehend genannten Ausführungsformen der Erfindung, dadurch gekennzeichnet, dass ≥ 4 und ≤ 12 Gew.-% der Sonnenschutzfilter-Substanzen Octocrylen sind.

Eine zwölfte bevorzugte Ausführungsform der Erfindung umfasst eine Sonnenschutz-Zusammensetzung gemäß einer der vorstehend genannten Ausführungsformen der Erfindung, dadurch gekennzeichnet, dass diese wenigstens eine öllösliche Sonnenschutzfilter-Substanz enthält.

Eine dreizehnte bevorzugte Ausführungsform der Erfindung umfasst eine Sonnenschutz-Zusammensetzung gemäß einer der vorstehend genannten Ausführungsformen der Erfindung, dadurch gekennzeichnet, dass diese ≥ 5 und ≤ 20 Gew.-% an öllösliche Sonnenschutzfilter-Substanz enthält.

Die vorliegende Erfindung wird an Hand der folgenden Beispiele erläutert.

### Beispiele:

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.

Die Bestimmung der Festkörpergehalte (nicht-flüchtiger Anteil) erfolgte nach DIN-EN ISO 3251.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Das zahlenmittlere Molekulargewicht wurde bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2xPSS SDV linear M, 8x300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Die Trübungswerte [NTU] wurden durch eine Streulichtmessung im 90°-Winkel (Nephelometrie) bei einer Wellenlänge der Mess-Strahlung 860 nm entsprechend DIN EN ISO 7027 bestimmt, durchgeführt bei 23°C mit einem Labortrübungsmessgerät Modell 2100AN der Firma HACH LANGE GmbH, Berlin, Deutschland.

### Verwendete Substanzen und Abkürzungen:

- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlenmittleres zahlenmittleres Molekulargewicht 1000 g/mol (BASF AG, Ludwigshafen, DE)
- Ethanol: Soweit nicht anderweitig gekennzeichnet wurde mit MEK vergälltes Ethanol der Fa. Nordbrand, Norhausen, DE eingesetzt.

Isocyanate und die weiteren polymeren Polyole wurden eingesetzt von der Bayer MaterialScience AG, Leverkusen, DE. Weitere Chemikalien von Sigma-Aldrich Chemie GmbH, Taufkirchen, DE. Die Rohstoffe wurden, soweit nicht anders erwähnt, ohne weitere Reinigung oder Vorbehandlung eingesetzt.

### Beispiel 1: Herstellung einer Polyurethan-Lösung in Ethanol (erfindungsgemäß)

150 g PolyTHF^{®} 2000 und 37,50 g PolyTHF^{®} 1000 wurden in einer Standard-Rührapparatur für eine Stunde bei 100°C im Membranpumpenvakuum entwässert und anschließend unter Stickstoff bei 80°C vorgelegt. Dann wurde bei 80°C innerhalb von 5 min 75,06 g Isophorondiisocyanat zugegeben und es wurde bei 110°C so lange gerührt (ca. 3 Stunden), bis der theoretische NCO-Wert unterschritten war. Das Prepolymer wurde auf 40°C abgekühlt und es wurde in 630,4 g Ethanol (vergällt mit Diethylphthalat) gelöst und anschließend wurde die Temperatur auf 15°C reduziert. Dann wurde eine Lösung aus 37,6 g Methylenbis(4-aminocyclohexan) und 270 g Ethanol (vergällt mit Diethylphthalat) innerhalb von 30 min zudosiert: nach weitere 30 Minuten bei 20°C waren IR-spektroskopisch noch Isocyanatgruppen nachweisbar. Es wurde weiter für ca. 16 Stunden bei 23°C gerührt, bis IR-spektroskopisch keine freien Isocyanatgruppen mehr feststellbar waren.

Die erhaltene klare, lagerstabile Lösung hatte nachfolgende Eigenschaften:

| | |
|---|---|
| Feststoffgehalt: | 23 % |
| Viskosität (Viskosimeter, 23°C): | 280 mPas |
| Trübungswert: | 1,2 NTU |

### Beispiel 2: Herstellung einer Polyurethan-Lösung in Ethanol (erfindungsgemäß)

300 g PolyTHF^{®} 1000 wurden in einer Standard-Rührapparatur für eine Stunde bei 100°C im Membranpumpenvakuum entwässert und anschließend unter Stickstoff bei 80°C vorgelegt. Dann wurde bei 80°C innerhalb von 5 min 133,44 g Isophorondiisocyanat zugegeben und es wurde bei 110°C so lange gerührt (ca. 3 Stunden), bis der theoretische NCO-Wert unterschritten war. Das Prepolymer wurde auf 40°C abgekühlt und es wurde in 517 g Ethanol (vergällt mit MEK) gelöst und anschliessend wurde die Temperatur auf 16°C reduziert. Dann wurde eine Lösung aus 58,8 g Methylenbis(4-aminocyclohexan) und 222 g Ethanol (vergällt mit MEK) innerhalb von 30 min zudosiert, danach wurden weitere 410 g Ethanol zugesetzt. Es wurde weiter gerührt, bis IR-spektroskopisch keine freien Isocyanatgruppen mehr feststellbar waren.

Die erhaltene klare, lagerstabile Lösung hatte nachfolgende Eigenschaften:

| | |
|---|---|
| Feststoffgehalt: | 30,2 % |
| Viskosität (Viskosimeter, 23°C): | 85000 mPas |

### Beispiel 3: Herstellung einer Polyurethan-Lösung in Ethanol (erfindungsgemäß)

211 g PolyTHF^{®} 2000 und 52,7 g PolyTHF^{®} 1000 wurden in einer Standard-Rührapparatur für eine Stunde bei 100°C im Membranpumpenvakuum entwässert, dann wurden 5,4 g Neopentylglykol zugesetzt und die Mischung wurde anschließend unter Stickstoff bei 80°C vorgelegt. Dann wurde bei 80°C innerhalb von 5 min 93,4 g Isophorondiisocyanat zugegeben und es wurde bei 110°C so lange gerührt (ca. 3 Stunden), bis der theoretische NCO-Wert unterschritten war. Das Prepolymer wurde auf 40°C abgekühlt und es wurde in 420 g Ethanol (vergällt mit Diethylphthalat) gelöst und anschliessend wurde die Temperatur auf 17°C reduziert. Dann wurde eine Lösung aus 35,3 g Methylenbis(4-aminocyclohexan) und 180 g Ethanol (vergällt mit Diethylphthalat) innerhalb von 30 min zudosiert. Es wurden weitere 0,67 g Methylenbis(4-aminocyclohexan) zugesetzt und dann weiter gerührt, bis IR-spektroskopisch keine freien Isocyanatgruppen mehr feststellbar waren.

Die erhaltene klare, lagerstabile Lösung hatte nachfolgende Eigenschaften:

| | |
|---|---|
| Feststoffgehalt: | 40,5 % |
| Viskosität (Viskosimeter, 23°C): | 7060 mPas |

### Beispiel 4: Herstellung einer Polyurethan-Lösung in Ethanol (erfindungsgemäß)

226,2 g Polypropylenglykol mit einer zahlenmittleren Molekulargewicht von 2000 g/mol und 62,5 g Polypropylenglykol mit einer zahlenmittleren Molekulargewicht von 1000 g/mol wurden in einer Standard-Rührapparatur für eine Stunde bei 100°C im Membranpumpenvakuum entwässert, die Mischung wurde anschließend unter Stickstoff bei 80°C vorgelegt. Dann wurde bei 80°C innerhalb von 5 min 83,4 g Isophorondiisocyanat zugegeben und es wurde bei 6 Stunden bei 120°C gerührt, bis der theoretische NCO-Wert unterschritten war. Das Prepolymer wurde auf 40°C abgekühlt und es wurde in 280 g Ethanol gelöst und anschließend wurde die Temperatur auf 18°C reduziert. Dann wurde eine Lösung aus 34,1 g Methylen-bis(4-aminocyclohexan) und 120 g Ethanol innerhalb von 30 min zudosiert. Es wurden weitere 4,5 g Methylenbis(4-aminocyclohexan) zugesetzt und dann weiter gerührt, bis IR-spektroskopisch keine freien Isocyanatgruppen mehr feststellbar waren.

Die erhaltene klare, lagerstabile Lösung hatte nachfolgende Eigenschaften:

| | |
|---|---|
| Feststoffgehalt: | 49,8 % |
| Viskosität (Viskosimeter, 23°C): | 1100 mPas |

### Beispiel 5: Herstellung einer Polyurethan-Lösung in Ethanol (erfindungsgemäß)

160 g PolyTHF^{®} 2000 und 40,0 g PolyTHF^{®} 1000 wurden in einer Standard-Rührapparatur für eine Stunde bei 100°C im Membranpumpenvakuum entwässert und anschließend unter Stickstoff bei 80°C vorgelegt. Dann wurde bei 80°C innerhalb von 5 min 62,9 g Bis-(4,4'-isocyanatocyclo¬hexyl)¬methan zugegeben und es wurde bei 110°C so lange gerührt (ca. 3 Stunden), bis der theoretische NCO-Wert unterschritten war. Das Prepolymer wurde auf 40°C abgekühlt und es wurde in 595 g Ethanol gelöst und anschließend wurde die Temperatur auf 19°C reduziert. Dann wurde eine Lösung aus 20,2 g Methylenbis(4-aminocyclohexan) und 255 g Ethanol innerhalb von 30 min zudosiert. Es wurden weitere 4,5 g Methylenbis(4-aminocyclohexan) zugesetzt und dann weiter gerührt, bis IR-spektroskopisch keine freien Isocyanatgruppen mehr feststellbar waren.

Die erhaltene klare, lagerstabile Lösung hatte nachfolgende Eigenschaften:

| | |
|---|---|
| Feststoffgehalt: | 25,2 % |
| Viskosität (Viskosimeter, 23°C): | 3400 mPas |

### Vergleichsbeispiel 1: Versuch zur Herstellung einer Polyurethanharnstoff-Lösung in Ethanol

100 g PolyTHF^{®} 2000, 25,0 g PolyTHF^{®} 1000 und 127,5 g eines linearen, difunktionellen, amorphen Polyestersdiols auf Adipinsäure, 1,6-Hexandiol und Neopentlyglykol mit einer zahlenmittleren Molekulargewicht von 1700 g/mol wurden in einer Standard-Rührapparatur für eine Stunde bei 100°C im Membranpumpenvakuum entwässert und anschließend unter Stickstoff bei 80°C vorgelegt. Dann wurde bei 80°C innerhalb von 5 min 66,7 g Isophorondiisocyanat zugegeben und es wurde bei 110°C so lange gerührt (ca. 3 Stunden), bis der theoretische NCO-Wert unterschritten war. Das Prepolymer wurde auf 40°C abgekühlt und es wurde in 720 g Ethanol gelöst, wobei sich das Produkt nicht vollständig löste und anschließend wurde die Temperatur auf 17°C reduziert. Dann wurde eine Lösung aus 25,2 g Methylenbis(4-aminocyclohexan) und 310 g Ethanol innerhalb von 30 min zudosiert, dabei entstand eine weisse Trübung. Dann wurde weiter gerührt, wobei sich keine stabile Lösung bildete sondern sich ein 2 phasiges Gemisch ergab aus dem sich die feste Phase absetzte.

### Vergleichsbeispiel 2: Versuch zur Herstellung einer Polyurethanharnstoff-Lösung in Ethanol

200 g eines linearen, difunktionellen, Polycarbonatdiols auf Basis von 1,6-Hexandiol, mit einer zahlenmittleren Molekulargewicht von 2000 g/mol und 50 g eines linearen, difunktionellen, Polycarbonatdiols auf Basis von 1,6-Hexandiol, mit einer zahlenmittleren Molekulargewicht von 1000 g/mol wurden in einer Standard-Rührapparatur für eine Stunde bei 100°C im Membranpumpenvakuum entwässert und anschließend unter Stickstoff bei 80°C vorgelegt. Dann wurde bei 80°C innerhalb von 5 min 66,7 g Isophorondiisocyanat zugegeben und es wurde bei 110°C so lange gerührt (ca. 3 Stunden), bis der theoretische NCO-Wert unterschritten war. Das Prepolymer wurde auf 40°C abgekühlt und es wurde in 720 g Ethanol gelöst, wobei sich das Produkt nicht vollständig löste und anschließend wurde die Temperatur auf 17°C reduziert. Dann wurde eine Lösung aus 25,2 g Methylenbis(4-aminocyclohexan) und 310 g Ethanol innerhalb von 30 min zudosiert, dabei entstand eine zweiphasige Mischung. Dann wurde weiter gerührt, wobei sich keine stabile Lösung bildete sondern sich ein 2 phasiges Gemisch ergab aus dem sich die feste Phase absetzte.

### Kompatibilität mit Ethanol

Es wurde die Kompatibilität der erfindungsgemäß hergestellten Polyurethanharnstofflösungen mit Ethanol im Vergleich zu Polyurethan basierten Filmbildnern nach dem Stand der Technik geprüft. Die Polyurethane, die in den Filmbildnern nach dem Stand der Technik eingesetzt wurden, weisen ionisch hydrophilierende Gruppen auf. Dazu wurde bei 23 °C jeweils eine Mischung aus Ethanol und dem Filmbildner hergestellt, die 2 Gew.-% Aktivstoff (jeweiliges Polyurethan) enthielten und die Trübungswerte der Mischungen bestimmt.

**Tabelle 1:**

| **Handelsname** | **INCI-Name** | **Lieferform** | **Feststoffgehalt [%]** | **Aussehen** | **Tübungswert [NTU]** |
|---|---|---|---|---|---|
| Baycusan^{®} C 1000 (BMS AG) | Polyurethane-34 | Wässrige, ionisch hydrophilierte Poly-urethandispersion | 40 | milchig, leichtes Sediment | 1410 |
| Baycusan^{®} C 1008 (BMS AG) | Polyurethane-48 | Wässrige, ionisch hydrophilierte Poly-urethandispersion | 30 | leicht bläulich, kolloidal | 500 |
| Erfindungsgemäße Polyurethanharnstofflösung | - | ethanolische Lösung | 23 | Klar | 3,5 |
| Avalure^{®} U 450 (Lubrizol) | PPG-17/IPDI/DMPA Copolymer | Wässrige, ionisch hydrophilierte Polyurethandispersion | 38 | leicht bläulich, kolloidal | 102 |

### Anwendungstechnische Vergleichsversuche:

Sofern nicht abweichend gekennzeichnet, beziehen sich alle Prozentangaben auf das Gewicht.

Aus den in Tabelle 2 angegebenen Bestandteilen wurde durch Vermischen der Bestandteile eine kosmetische Zusammensetzung mit Sonnenschutzeigenschaften hergestellt. Dazu wurden die Komponenten der Ölphase, außer Cyclopentasiloxan und Caprylylmethicon, vermischt und so lange auf 80°C erhitz, bis die Phase klar war. Anschließend wurde die Ölphase auf 45°C abgekühlt und Cyclopentasiloxan und Caprylylmethicon zugegeben. Die Komponenten der Ethanolphase wurden zusammengegeben und bei 23°C gerührt bis ein homogenes Gemisch entstanden war. Dann wurde die Ethanolphase zur Ölphase gegeben und die beiden Phasen bei 23°C verrührt bis ein homogenes Gemisch entstanden war.

Zum Vergleich wurde dieselbe Formulierung ohne die erfindungsgemäße Polyurethanharnstofflösung hergestellt.

Zum weiteren Vergleich wurde die gleiche Zusammensetzung formuliert, jedoch die erfindungsgemäße Polyurethanharnstofflösung durch andere Filmbildner wie Dermacryl^{®} 79, Luviskol^{®} K-90, Avalure^{®} U 450 oder Baycusan ^{®} C1000 oder C1008 ersetzt. Dabei wurden alle Komponenten in denselben Mengen eingesetzt und die Menge der Filmbildnerkomponente je nach Konzentration so gewählt, dass die Formulierung 2 Gew.-% an polymerem Aktivstoff enthält.

**Tabelle 2:**

| **Phase** | **Bestandteile** | **[Gew.-%]** |
|---|---|---|
| Ethanolphase | Ethanol | 47,70 |
| | Erfindungsgemäße ethanolische Polyurethanharnstofflösung (Polyurethanharnstoff als Aktivstoff) | 8,70 (2,00) |
| | Butyleneglycol | 2,50 |
| Ölphase | 4-(t-Butyl)-4'-methoxydibenzoylmethan | 4,00 |
| | Homosalat | 7,00 |
| | Octocrylen | 7,00 |
| | bis-Ethylhexyloxyphenolmethoxyphenyltriazin | 2,00 |
| | Ethylhexylsalicylat | 5,00 |
| | C12-15 Alkylbenzoat | 4,40 |
| | Dicaprylylcarbonat | 5,80 |
| | Tridecylsalicylate | 2,90 |
| | Cyclopentasiloxane | 1,50 |
| | Caprylylmethicone | 1,50 |
| | **Summe** | 100,00 |

Tabelle 3 gibt die Eigenschaften der erhaltenen kosmetischen Zusammensetzungen wieder.

**Tabelle 3:**

| **Handelsname** | **INCI-Name** | **Lieferform** | **Feststoffgehalt [%]** | **Formulierungsaussehen** | **Tübungswert [NTU],** |
|---|---|---|---|---|---|
| Baycusan^{®} C 1000 (Bayer MaterialScience AG) | Polyurethane-34 | Wässrige PUD | 40 | Milchig | Nicht messbar |
| Baycusan^{®} C 1008 (Bayer MaterialScience AG) | Polyurethane-48 | Wässrige PUD | 30 | Milchig | Nicht messbar |
| Erfindungsgemäße Polyurethanharnstofflösung | - | ethanolische Lösung | 23 | Klar | 3,5 |
| Avalure^{®} U 450 (Lubrizol) | PPG-17/IPDI/DMPA Copolymer | Wässrige PUD | 38 | Instabil | - |
| Luviskol^{®} K 90 (BASF) | PVP | Feststoff | 100 | Instabil | - |
| Dermacryl^{®} 79 (Akzo Nobel) | Acrylat/Octylacrylamid-Copolymer | Feststoff | 100 | Klar | 3,9 |

### Ergebnisse der in vivo SPF-Messung

Zu den klaren Formulierungen wurden die SPF-Werte in Vivo bestimmt. Die Ergebnisse sind in Tab. 3 zusammengefasst. Die ermittelten Werte zeigen eindeutig den hohen SPF-Boosting-Effekt der erfindungsgemäßen Sonnenschutz-Zusammensetzung.

Die In-Vivo-Messung des SPF erfolgte gemäß ISO 24444:2010 auf 5 Probanden.

**Tabelle 4:**

| SPF ohne Polyurethanharnstofflösung | SPF mit erfindungsgemäßer Polyurethanharnstofflösung | SPF mit Acrylat/Octylacrylamid- Copolymer Dermacryl^{®} 79 |
|---|---|---|
| 11,4 | 31,2 | 24,1 |

## Patentansprüche

1. Verfahren zur Herstellung einer kosmetischen Zusammensetzung, **dadurch gekennzeichnet, dass** wenigstens ein Polyurethanharnstoff, welcher keine ionisch hydrophilierenden Gruppen aufweist und welcher gelöst in einem Lösemittel oder Lösemittelgemisch ist, eingesetzt wird, wobei das Lösemittel ausschließlich aus einem oder mehreren monohydroxyfunktionellen Alkoholen besteht oder ein Lösemittelgemisch ist, bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, die kosmetische Zusammensetzung einen Wasseranteil von ≥ 0 und ≤ 5 Gew.-% aufweist und wenigstens eine öllösliche organische Sonnenschutzfilter-Substanz enthält,
wobei der Polyurethanharnstoff aufgebaut ist aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b), wobei Mn durch Gelpermeationschromatographie (GPC) bestimmt wird.

2. Verfahren gemäß Anspruch 1, **dadurch gekennzeichnet, dass** der Polyurethanharnstoff keine hydrophilierenden Gruppen aufweist.

3. Verfahren gemäß Anspruch 1 oder 2, **dadurch gekennzeichnet, dass** die Komponente b) ausgewählt ist aus Poly(tetramethylenglykol)polyetherpolyolen.

4. Verfahren gemäß einem der Ansprüche 1 bis 3, **dadurch gekennzeichnet, dass** die Komponente b) eine zahlenmittlere Molekulargewicht Mn ≥ 500 und ≤ 2500 g/mol und eine Hydroxylfunktionalität von ≥ 1,9 und ≤ 3 aufweist.

5. Verfahren gemäß einem der Ansprüche 1 bis 4, **dadurch gekennzeichnet, dass** die Komponente a) ausgewählt ist aus aliphatischen, araliphatischen und/oder cycloaliphatischen Diisocyanaten, welche mindestens eine Isocyanatgruppe aufweisen, die an ein sekundäres und/oder tertiäres C-Atom gebunden ist.

6. Verfahren gemäß einem der Ansprüche 1 bis 5, **dadurch gekennzeichnet, dass** die Komponente a) ausgewählt ist aus IPDI und/oder H12-MDI.

7. Verfahren gemäß einem der Ansprüche 1 bis 6, **dadurch gekennzeichnet, dass** die Komponente c) ausgewählt ist aus Aminen, die mindestens zwei Aminogruppen aufweisen, die an primäre und/oder sekundäre C-Atome gebunden sind.

8. Verfahren gemäß einem der Ansprüche 1 bis 7, **dadurch gekennzeichnet, dass** die Komponente c) ausgewählt ist aus symmetrisch aufgebauten Diaminen.

9. Verfahren gemäß einem der Ansprüche 1 bis 8, **dadurch gekennzeichnet, dass** die Komponente c) ausgewählt ist aus Ethylendiamin und/oder H12-MDA.

10. Verfahren gemäß einem der Ansprüche 1 bis 9, **dadurch gekennzeichnet, dass** die monohydroxyfunktionellen Alkohole ausgewählt sind aus aliphatischen Alkohole mit einem bis sechs Kohlenstoffatomen.

11. Kosmetische Zusammensetzung erhältlich nach einem Verfahren gemäß einem der Ansprüche 1 bis 10, wobei die kosmetische Zusammensetzung eine alkoholische Lösung ist, einen Wasseranteil von ≥ 0 und ≤ 5 Gew.-% aufweist und wenigstens eine öllösliche organische Sonnenschutzfilter-Substanz enthält.

12. Verfahren zur Herstellung einer kosmetischen Beschichtung auf Nägeln und/oder keratinen Fasern unter Verwendung von kosmetischen Zusammensetzungen gemäß Anspruch 11, wobei die kosmetische Zusammensetzung auf Nägel und/oder keratinen Fasern aufgetragen wird.

13. Sonnenschutz-Zusammensetzung enthaltend wenigstens eine öllösliche organische Sonnenschutzfiltersubstanz und wenigstens einen Polyurethanharnstoff, welcher keine ionisch hydrophilierenden Gruppen aufweist und welcher gelöst in einem Lösemittel oder Lösemittelgemisch ist, wobei das Lösemittel ausschließlich aus einem oder mehreren monohydroxyfunktionellen Alkoholen besteht oder ein Lösemittelgemisch ist, bestehend aus ausschließlich organischen Lösemitteln, welches ≥ 50 Gew.-%, bezogen auf die Gesamtmasse des Lösemittelgemisches, an wenigstens einem monohydroxyfunktionellen Alkohol enthält, wobei die Zusammensetzung einen Wasseranteil von ≥ 0 und ≤ 5 Gew.-% aufweist und der Polyurethanharnstoff aufgebaut ist aus
a) wenigstens einem aliphatischen, araliphatischen und/oder cycloaliphatischen Diisocyanat,
b) wenigstens einem Polyetherpolyol mit einem zahlenmittleren Molekulargewicht Mn ≥ 400 und ≤ 6000 g/mol und einer Hydroxylfunktionalität von ≥ 1,5 und ≤ 4,
c) wenigstens einer aminofunktionellen Verbindung, die mindestens zwei isocyanatreaktive Aminogruppen aufweist,
d) gegebenenfalls wenigstens einem Alkohol, der mindestens zwei Hydroxylgruppen und eine Molmasse ≥ 60 und ≤ 399 g/mol aufweist,
e) gegebenenfalls wenigstens einer Verbindung die eine gegenüber Isocyanatgruppen reaktive Gruppe aufweist und
f) gegebenenfalls ≤ 20 Gew.-%, bezogen auf die Gesamtmasse des Polyurethanharnstoffs, an wenigstens einem Polyol mit einer zahlenmittleren Molekulargewicht Mn ≥ 500 und ≤ 6000 g/mol und einer Hydroxylfunktionalität von ≥ 1,5 und ≤ 4, welches unterschiedlich ist von b), wobei Mn durch Gelpermeationschromatographie (GPC) bestimmt wird.

## Claims

1. Process for producing a cosmetic composition, **characterized in that**
at least one polyurethaneurea which has no ionically hydrophilizing groups and has been dissolved in a solvent or solvent mixture is used, wherein the solvent consists exclusively of one or more monohydroxy-functional alcohols or is a solvent mixture consisting of exclusively organic solvents and containing ≥ 50% by weight, based on the total mass of the solvent mixture, of at least one monohydroxy-functional alcohol, and the cosmetic composition has a water content of ≥ 0% and ≤ 5% by weight and comprises at least one oil-soluble organic sunscreen filter substance,
wherein the polyurethaneurea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethaneurea, of at least one different polyol than b) having a number-average molecular weight Mn of ≥ 500 and ≤ 6000 g/mol and a hydroxyl functionality of ≥ 1.5 and ≤ 4, where Mn is determined by gel permeation chromatography (GPC).

2. Process according to Claim 1, **characterized in that** the polyurethaneurea does not have any hydrophilizing groups.

3. Process according to Claim 1 or 2, **characterized in that** component b) is selected from poly(tetramethylene glycol) polyether polyols.

4. Process according to any of Claims 1 to 3, **characterized in that** component b) has a number-average molecular weight Mn of ≥ 500 and ≤ 2500 g/mol and a hydroxyl functionality of ≥ 1.9 and ≤ 3.

5. Process according to any of Claims 1 to 4, **characterized in that** component a) is selected from aliphatic, araliphatic and/or cycloaliphatic diisocyanates having at least one isocyanate group bonded to a secondary and/or tertiary carbon atom.

6. Process according to any of Claims 1 to 5, **characterized in that** component a) is selected from IPDI and/or H12-MDI.

7. Process according to any of Claims 1 to 6, **characterized in that** component c) is selected from amines having at least two amino groups bonded to primary and/or secondary carbon atoms.

8. Process according to any of Claims 1 to 7, **characterized in that** component c) is selected from diamines of symmetric structure.

9. Process according to any of Claims 1 to 8, **characterized in that** component c) is selected from ethylenediamine and/or H12-MDA.

10. Process according to any of Claims 1 to 9, **characterized in that** the monohydroxy-functional alcohols are selected from aliphatic alcohols having one to six carbon atoms.

11. Cosmetic composition obtainable by a process according to any of Claims 1 to 10, wherein the cosmetic composition is an alcoholic solution having a water content of ≥ 0% and ≤ 5% by weight and comprising at least one oil-soluble organic sunscreen filter substance.

12. Process for producing a cosmetic composition on nails and/or keratinic fibers using cosmetic compositions according to Claim 11, wherein the cosmetic composition is applied to nails and/or keratinic fibers.

13. Sunscreen composition comprising at least one oil-soluble sunscreen filter substance and at least one polyurethaneurea which has no ionically hydrophilizing groups and has been dissolved in a solvent or solvent mixture, wherein the solvent consists exclusively of one or more monohydroxy-functional alcohols or is a solvent mixture consisting of exclusively organic solvents and containing ≥ 50% by weight, based on the total mass of the solvent mixture, of at least one monohydroxy-functional alcohol, wherein the composition has a water content of ≥ 0% and ≤ 5% by weight and the polyurethaneurea has been formed from
a) at least one aliphatic, araliphatic and/or cycloaliphatic diisocyanate,
b) at least one polyether polyol having a number-average molecular weight Mn of ≥ 400 and ≤ 6000 g/mol and a hydroxyl functionality of ≥ 1.5 and ≤ 4,
c) at least one amino-functional compound having at least two isocyanate-reactive amino groups,
d) optionally at least one alcohol having at least two hydroxyl groups and a molar mass of ≥ 60 and ≤ 399 g/mol,
e) optionally at least one compound having a group reactive toward isocyanate groups and
f) optionally ≤ 20% by weight, based on the total mass of the polyurethaneurea, of at least one different polyol than b) having a number-average molecular weight Mn of ≥ 500 and ≤ 6000 g/mol and a hydroxyl functionality of ≥ 1.5 and ≤ 4, where Mn is determined by gel permeation chromatography (GPC).

## Revendications

1. Procédé pour la préparation d'une composition cosmétique, **caractérisé en ce qu'**au moins une polyuréthaneurée qui ne présente aucun groupe hydrophilisant de manière ionique et qui est dissoute dans un solvant ou un mélange de solvants, est utilisée, le solvant étant exclusivement constitué d'un ou plusieurs alcools fonctionnalisés par monohydroxy ou étant un mélange de solvants constitué exclusivement de solvants organiques, lequel contient ≥ 50 % en poids, par rapport à la masse totale du mélange de solvants, d'au moins un alcool fonctionnalisé par monohydroxy, la composition cosmétique présentant une teneur en eau ≥ 0 et ≤ 5 % en poids et contient au moins une substance d'écran solaire organique soluble dans l'huile,
la polyuréthaneurée étant construite à partir
a) d'au moins un diisocyanate aliphatique, araliphatique et/ou cycloaliphatique,
b) d'au moins un polyétherpolyol doté d'un poids moléculaire moyen en nombre Mn ≥ 400 et ≤ 6 000 g/mole et d'une fonctionnalité d'hydroxyle ≥ 1,5 et ≤ 4,
c) d'au moins un composé fonctionnalisé par amino qui présente au moins deux groupes amino réactifs envers un isocyanate,
d) éventuellement d'au moins un alcool, qui présente au moins deux groupes hydroxyle et une masse molaire ≥ 60 et ≤ 399 g/mole,
e) éventuellement d'au moins un composé qui présente un groupe réactif envers des groupes isocyanate et
f) éventuellement de ≤ 20 % en poids, par rapport à la masse totale de la polyuréthaneurée, d'au moins un polyol doté d'un poids moléculaire moyen en nombre Mn ≥ 500 et ≤ 6 000 g/mole et d'une fonctionnalité d'hydroxyle de ≥ 1,5 et ≤ 4, qui est différent de b), Mn étant déterminé par chromatographie à perméation de gel (CPG).

2. Procédé selon la revendication 1, **caractérisé en ce que** la polyuréthaneurée ne présente aucun groupe hydrophilisant.

3. Procédé selon la revendication 1 ou 2, **caractérisé en ce que** le composant b) est choisi parmi des poly(tétraméthylèneglycol)polyétherpolyols.

4. Procédé selon l'une quelconque des revendications 1 à 3, **caractérisé en ce que** le composant b) présente un poids moléculaire moyen en nombre Mn ≥ 500 et ≤ 2 500 g/mole et une fonctionnalité d'hydroxyle ≥ 1,9 et ≤ 3.

5. Procédé selon l'une quelconque des revendications 1 à 4, **caractérisé en ce que** le composant a) est choisi parmi des diisocyanates aliphatiques, araliphatiques et/ou cycloaliphatiques qui présentent au moins un groupe isocyanate qui est lié à un atome de C secondaire et/ou tertiaire.

6. Procédé selon l'une quelconque des revendications 1 à 5, **caractérisé en ce que** le composant a) est choisi parmi l'IPDI et/ou le H12-MDI.

7. Procédé selon l'une quelconque des revendications 1 à 6, **caractérisé en ce que** le composant c) est choisi parmi des amines qui présentent au moins deux groupes amino qui sont liés à des atomes de C primaires et/ou secondaires.

8. Procédé selon l'une quelconque des revendications 1 à 7, **caractérisé en ce que** le composant c) est choisi parmi des diamines construites de manière symétrique.

9. Procédé selon l'une quelconque des revendications 1 à 8, **caractérisé en ce que** le composant c) est choisi parmi les éthylènediamines et/ou le H12-MDA.

10. Procédé selon l'une quelconque des revendications 1 à 9, **caractérisé en ce que** les alcools fonctionnalisés par monohydroxy sont choisis parmi des alcools aliphatiques comportant un à six atomes de carbone.

11. Composition cosmétique pouvant être obtenue par un procédé selon l'une quelconque des revendications 1 à 10, la composition cosmétique étant une solution alcoolique, présentant une teneur en eau ≥ 0 et ≤ 5 % en poids et contenant au moins une substance d'écran solaire organique soluble dans l'huile.

12. Procédé pour la préparation d'un revêtement cosmétique sur des ongles et/ou des fibres kératiniques en utilisant des compositions cosmétiques selon la revendication 11, la composition cosmétique étant appliquée sur des ongles et/ou des fibres kératiniques.

13. Composition d'écran solaire contenant au moins une substance d'écran solaire organique soluble dans l'huile et au moins une polyuréthaneurée, qui ne présente aucun groupe hydrophilisant de manière ionique et qui est dissoute dans un solvant ou un mélange de solvants, le solvant étant exclusivement constitué d'un ou plusieurs alcools fonctionnalisés par monohydroxy ou étant un mélange de solvants constitué exclusivement de solvants organiques, lequel contient ≥ 50 % en poids, par rapport à la masse totale du mélange de solvants, d'au moins un alcool fonctionnalisé par monohydroxy, la composition présentant une teneur en eau ≥ 0 et ≤ 5 % en poids et
la polyuréthaneurée étant construite à partir
a) d'au moins un diisocyanate aliphatique, araliphatique et/ou cycloaliphatique,
b) d'au moins un polyétherpolyol doté d'un poids moléculaire moyen en nombre Mn ≥ 400 et ≤ 6 000 g/mole et d'une fonctionnalité d'hydroxyle de ≥ 1,5 et ≤ 4,
c) d'au moins un composé fonctionnalisé par amino qui présente au moins deux groupes amino réactifs envers un isocyanate,
d) éventuellement d'au moins un alcool, qui présente au moins deux groupes hydroxyle et une masse molaire ≥ 60 et ≤ 399 g/mole,
e) éventuellement d'au moins un composé qui présente un groupe réactif envers des groupes isocyanate et
f) éventuellement de ≤ 20 % en poids, par rapport à la masse totale de la polyuréthaneurée, d'au moins un polyol doté d'un poids moléculaire moyen en nombre Mn ≥ 500 et ≤ 6 000 g/mole et d'une fonctionnalité d'hydroxyle ≥ 1,5 et ≤ 4, qui est différent de b), Mn étant déterminé par chromatographie à perméation de gel (CPG).
